# EUROPÄISCHE PATENTSCHRIFT

(11) **EP 3 230 300 B1**
(45) Veröffentlichungstag und Bekanntmachung des Hinweises auf die Patenterteilung: **24.11.2021**
(21) Anmeldenummer: 15808218.0
(22) Anmeldetag: 11.12.2015
(51) Int. Cl.: C07D 519/00, A61P 35/00, A61K 31/55

(54) **INHIBITOREN ZUR HEMMUNG DER TUMORMETASTASIERUNG**
INHIBITORS FOR INHIBITION OF TUMOR METASTASIS
INHIBITEURS POUR L'INHIBITION DE LA MÉTASTASE DES TUMEURS

(30) Priorität: 11.12.2014 DE 102014118413
(43) Veröffentlichungstag der Anmeldung: 18.10.2017
(73) Patentinhaber: Forschungsverbund Berlin E.V., 12489 Berlin (DE); Universität zu Köln, 50923 Köln (DE)
(72) Erfinder: KÜHNE, Ronald, 12683 Berlin (DE); SCHMALZ, Hans-Günther, 50321 Brühl (DE); MÜLLER, Matthias, 10559 Berlin (DE); REUTER, Cedric, 50674 Köln (DE); SOICKE, Arne, 51491 Overath (DE); OPITZ, Robert, 12349 Berlin (DE); BARONE, Matthias, 10439 Berlin (DE); OSCHKINAT, Hartmut, 10437 Berlin (DE)
(74) Vertreter: Hertin und Partner Rechts- und Patentanwälte PartG mbB
(86) Internationale Anmeldenummer: PCT/EP2015/079410
(87) Internationale Veröffentlichungsnummer: WO 2016/092069

(56) Entgegenhaltungen:
- WO-A1-98/54208
- WO-A1-2008/040332
- WO-A1-2013/030111
- ROBERT OPITZ ET AL: "A modular toolkit to inhibit proline-rich motif-mediated protein-protein interactions", PROCEEDINGS OF THE NATIONAL ACADEMY OF SCIENCES, Bd. 112, Nr. 16, 6. April 2015 (2015-04-06), Seiten 5011-5016, XP055248309, US ISSN: 0027-8424, DOI: 10.1073/pnas.1422054112

## Beschreibung

Die vorliegende Erfindung betrifft chemische Verbindungen, die insbesondere als Struktur-Mimetika prolinreicher Peptide eingesetzt werden können. Die Verbindungen der vorliegenden Erfindung sind in der Lage ena/VASP-EVH1-vermittelte Protein-Protein-Wechselwirkungen selektiv zu hemmen. Die Erfindung betrifft weiterhin die Verwendung dieser Verbindungen als pharmazeutische Wirkstoffe sowie die Verwendung der pharmazeutischen Wirkstoffe zur Behandlung von Tumorerkrankungen. Die chemischen Verbindungen der vorliegenden Erfindung können die Chemotaxis und Motilität invasiver Tumorzellen stark hemmen und somit in der Behandlung und/oder Prävention von Tumormetastasen eingesetzt werden.

### HINTERGRUND ZUR ERFINDUNG

Bei fast sämtlichen physiologischen und biochemischen Prozessen in lebenden Organismen spielen Wechselwirkungen zwischen Proteinen eine zentrale Rolle. Proteine erfüllen nicht nur biokatalytische Aufgaben (zum Beispiel als Enzym) sondern sind an verschiedenen biologischen Prozessen beteiligt. Eine selektive Hemmung der körperlichen Interaktion zwischen Peptidliganden und den entsprechenden Proteinrezeptoren kann einen wesentlichen Einfluss auf verschiedene biologische Mechanismen in der Zelle haben.

Es besteht daher im Stand der Technik das Bedürfnis, insbesondere bei der Behandlung von Krankheiten, Moleküle zu entwickeln, die spezifische Protein-Protein-Wechselwirkungen hemmen, um biologisch bzw. medizinisch relevante technische Effekte zu erzielen.

Ein Beispiel für solche Inhibitoren sind Struktur-Mimetika von Diprolin-Einheiten. Im Vergleich zu anderen Aminosäuren nimmt Prolin auf Basis seiner chemischen Struktur eine Sonderrolle ein. Prolin ist die einzige sekundäre, proteinogene Aminosäure die nach Knüpfung einer Peptidbindung über kein freies NH-Proton zur Ausbildung von Wasserstoffbrückenbindungen verfügt. Aus diesem Grund sind Polyprolin-Sequenzen nicht in der Lage klassische alpha-Helix oder beta-Faltblatt Sekundärstrukturen einzunehmen. Aufgrund dieser besonderen physikalischen Eigenschaften sind Polyprolin-Aminosäuresequenzen (z. B. prolinreiche Motive (PRM)) sehr gut geeignet, spezifische Protein-Protein-Wechselwirkungen mit Rezeptoren (z. B. prolinreiche Motive-bindende Domänen (PBD)) zu formieren. Durch die Bereitstellung von Polyprolin-Mimetika können einige dieser Wechselwirkungen selektiv gehemmt werden.

Einige beta-turn-Peptidmimetika sind als Modulatoren von Protein-Protein-Interaktionen im Stand der Technik bekannt. Die WO 2006/067091 A1 offenbart verschiedene Peptide und Peptidmimetika, welche die Homodimerisierung von MyD88 sowie die Interaktion zwischen MyD88 und TIR verhindern. Beta-turn-Peptidmimetika sind ebenfalls als Modulatoren von Protein-Protein-Interaktionen zwischen SH3-Domänen bekannt. Es wird beispielsweise in der WO 98/54208 offenbart, dass beta-turn-Peptidmimetika, welche Polyprolin-Motive und eine alpha-Helix-Struktur aufweisen, mit SH3-Domänen interagieren können. Witter et al (Bioorg. & Med. Chem. Let. 8 (1998) 3137) und Vartak et al (Organic Let. 2006, 8:5, 983) offenbaren verschiedene beta-turn-Peptidmimetika, die als Mimetika für eine Polyprolin-Sequenz eingesetzt werden können.

In WO 2008/040332 und in WO 2013/030111 sind weitere chemische Verbindungen offenbart, die als Diprolin-Mimetika funktionieren. Beispielsweise werden in WO 2013/030111 Peptidverbindungen offenbart, die zwei aneinandergrenzende Diprolin-Mimetika anstelle von vier Prolin-Aminosäuren aufweisen, wobei die Diprolin-Mimetika von Peptidsequenzen flankiert sind. Die darin beschriebenen Peptide zeigen eine gute Affinität zu einer VASP-EVH1-Domäne, sind jedoch auf Basis ihrer besonderen Peptid-Struktur aufwändig herzustellen. Die Peptidstrukturen werden weiterhin aufgrund der Peptidstruktur und der Anwesenheit von endogenen Proteasen *in vivo* abgebaut. Einige dieser Verbindungen zeigen ebenfalls eine sub-optimale Zellpermeabilität, die für die Anwendung als Medikament durchaus nachteilhaft ist.

Obwohl die darin offenbarten Verbindungen Protein-Protein-Wechselwirkungen durch selektive Bindung an einen Rezeptor inhibieren können, besteht weiterhin das Bedürfnis, weitere Polyprolin-Mimetika bereitzustellen, die verbesserte Bindungseigenschaften und entsprechende biologische Wirkungen aufweisen.

### DETAILLIERTE DARSTELLUNG DER ERFINDUNG

Im Lichte des Standes der Technik war es daher Aufgabe der Erfindung, Verbindungen bereitzustellen, die als Mimetika für polyprolinreiche Peptide verwendet werden können, insbesondere als pharmazeutische Wirkstoffe für die Behandlung von verschiedenen Krankheiten. Eine weitere Aufgabe der Erfindung war die Bereitstellung von neuartigen Krebsmedikamenten, die effektiv gegen die Invasion und Motilität von Krebszellen wirken.

Das erfindungsgemäße Problem wird gelöst durch die Bereitstellung einer Verbindung gemäß Formel I: 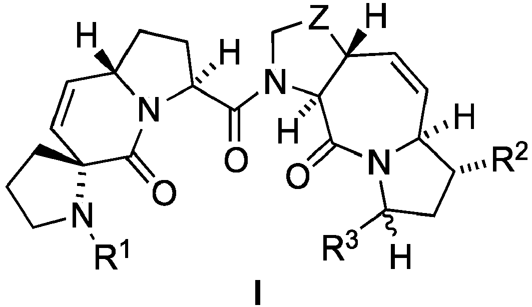 wobei
R1: H, Acyl, Peptidyl, Sulfonyl, Alkyl, Aryl oder Heteroaryl ist, wobei die Acyl, Peptidyl, Sulfonyl, Alkyl, Aryl oder Heteroaryl Substituenten gemäß R1 optional substituiert sind;
R2: CH3 oder C2H5;
R3: Alkyl, Acyl, Carbonsäure gemäß C(=O)X, wobei X = OH, O-Alkyl, O-Aryl, NRR', mit R, R' = H oder Alkyl, NROR', mit R, R' = H oder Alkyl, oder Heteroaryl;
Z: CH2 oder CH2CH2 ist.

Die optionale Substitution bezieht sich auf mögliche weitere Restgruppen, die an den genannten Restgruppen vorhanden sein können. Mögliche optionale Substitutionen können vorzugsweise Halogen, Acyl, Carboxyl, Amino, Carbamoyl, OH, O-alkyl, SH, S-alkyl, Alkyl, Aryl oder Heteroaryl sein. Wenn zum Beispiel R¹ Alkyl wäre, könnte ein substituiertes Alkyl ein Fluoralkyl sein. Bevorzugte Heteroaryl-Substituenten sind Imidazol, Triazol, Tetrazol oder Oxazol.

Es war überraschend, dass die erfindungsgemäßen Verbindungen - in einigen Ausführungsformen ohne flankierende Peptidstrukturen - eine gute Affinität zum Target, eine hohe Zellpermeabilität und hohe Stabilität aufweisen. Es war im Lichte des Standes der Technik nicht zu erwarten, dass die Abwesenheit einer flankierenden Peptidstruktur an der R³ Position keine negativen Einflüsse auf die Affinität des Moleküls zum Target haben würde. Die Verbesserungen der Affinität durch die Abwesenheit der flankierenden Peptidstrukturen an der R³-Position waren überraschend. Die erfindungsgemäßen Verbindungen zeigen ebenfalls eine überraschend gute Aktivität gegen die Motilität und Invasion von Krebszellen und stellen somit effektive Antikrebsreagenzien dar, die für eine klinische Anwendung geeignet sind.

Bevorzugt ist die stereochemische Konfiguration an dem Rest R³ wie folgt zu definieren (Formel I-a):

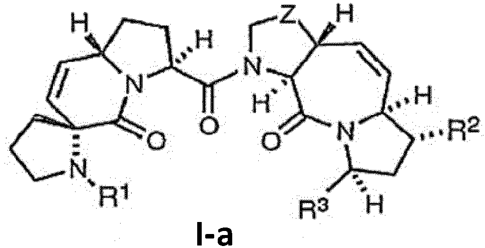

Die Substituenten für R¹, R², R³, Z sind für Formel I-a wie für Formel I (oben) zu definieren.

Es war überraschend, dass die erfindungsgemäßen Verbindungen nicht die Nachteile des Standes der Technik aufweisen. Die erfindungsgemäßen Verbindungen weisen verbesserte Eigenschaften auf, im Vergleich zu den bereits bekannten Verbindungen, zum Beispiel eine verbesserte Zellgängigkeit und daher erhöhte Bioverfügbarkeit. Die metabolische Stabilität der erfindungsgemäßen Verbindungen ist erhöht und die Stärke der Bindung am Target-Molekül sowie die hohe Selektivität für das Target-Molekül stellen weitere Vorteile der erfindungsgemäßen Verbindungen dar.

Eine bevorzugte Ausführungsform der vorliegenden Erfindung betrifft eine Verbindung gemäß der allgemeinen Formel II: 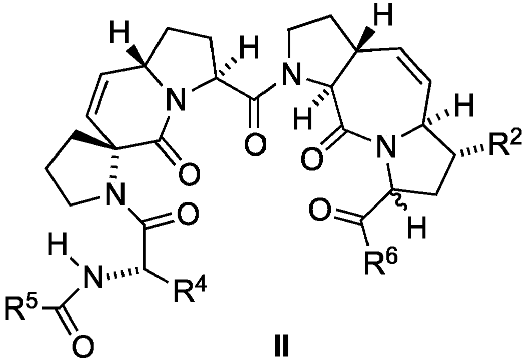 wobei
R2: CH3 oder C2H5;
R4: H, Alkyl, Cycloalkyl, Aryl, CH2-Aryl, CH2-Heteroaryl, oder ein heterozyklischer Substituent;
R5: H, Alkyl oder ein substituierter Alkylrest;
R6: OH, O-Alkyl, O-Aryl, NRR' mit R, R' = H oder alkyl, NROR' mit R, R' = H oder Alkyl, oder Heteroaryl.

Bevorzugt ist die stereochemische Konfiguration an dem Rest COR⁶ wie folgt zu definieren (Formel II-a):

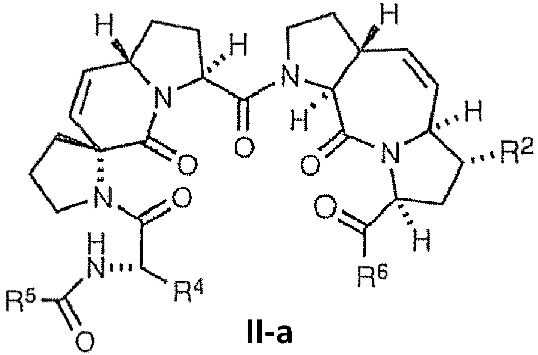

Die Substituenten R², R⁴, R⁵, R⁶ sind für Formel II-a wie für Formel II (oben) zu definieren.

Die erfindungsgemäßen Verbindungen gemäß Formel I, I-a, II oder II-a sind dadurch gekennzeichnet, dass R²: CH₃ oder C₂H₅ ist. Diese Substituenten ermöglichen eine besonders starke Affinität zum Target. Durch die Anwendung von Methyl oder Ethyl an der R²-Position kann das Molekül ideal in der Bindungstasche des Targets andocken und somit eine viel höhere Affinität verleihen.

Es ist weiterhin bevorzugt, dass der Substituent an der R²-Position die in der grafischen Darstellung der Formel gezeigte Stereochemie aufweist. Diese Stereochemie führt zu einer besonders hohen Affinität.

In einer bevorzugten Ausführungsform ist die erfindungsgemäße Verbindung gemäß Formel I, I-a, II oder II-a dadurch gekennzeichnet, dass R⁴: CH₂-aryl ist, insbesondere substituierte -CH₂-Phenyl, wobei die Substituent an der Ortho-Position der Phenylgruppe positioniert ist, wobei der Substituent bevorzugt Halogen ist, insbesondere Cl.

Bevorzugt an der R⁴-Position ist ein Substituent positioniert, der eine 2-Cl-Phenylalanin-Struktur im Molekül (an der R¹-Position der Formel I oder I-a) bildet. Verschiedene Halogene oder Ringstrukturen können eingesetzt werden. Als Halogen werden bevorzugt Cl, F und Br eingesetzt. Methyl oder andere bevorzugt kurzkettige Alkylgruppen (z. B. C₁-C₅) können ebenfalls als Substituent an der Ortho-Position der Phenylgruppe verwendet werden.

In einer bevorzugten Ausführungsform ist die erfindungsgemäße Verbindung gemäß Formel II oder II-a dadurch gekennzeichnet, dass R⁶: O-Alkyl, O-Aryl, NRR' mit R, R' = H oder alkyl, NROR' mit R, R' = H oder Alkyl oder Heteroaryl ist. Diese Substituenten zeigen den Vorteil, dass Sie eine viel höhere Zellpermeabilität verleihen, sodass größere Mengen des aktiven Wirkstoffes in die Zelle aufgenommen werden können.

Die Ester-Gruppen an der R⁶-Position können auch unter Umständen durch endogene Enzyme nach der Verabreichung in vivo gespalten werden, so dass eine Carboxylgruppe an der R6 Position vorhanden ist. Die Anwesenheit der freien Carboxylgruppe an der R⁶-Position (R⁶: OH) ermöglicht eine gute Affinität gegen das Target-Protein, wirkt aber gegen die Zellpermeabilität des Moleküls. Durch die Veresterung an dieser Position können "Pro-Drugs" erstellt werden, die eine gute Zellpermeabilität und Affinität zum Target zeigen, aber nach der Spaltung des Moleküls in vivo eine bessere Bindung und somit eine bessere Wirkung hervorrufen können.

Die Ethylester (R⁶: O-Et) werden relativ schnell in vivo gespalten, wobei die Methylester (R⁶: O-Me) und das Amid (R⁶: NH₂) eine besonders gute in vivo Stabilität zeigen.

Die Anwesenheit der NH₂-Gruppe an der R6-Position zeigt auch eine unerwartet gute Zellpermeabilität und Affinität zum Target.

In einer bevorzugten Ausführungsform ist die erfindungsgemäße Verbindung gemäß Formel II oder IIa dadurch gekennzeichnet, dass R⁶: O-CH3, O-C2H5 oder NH2 ist. Insbesondere diese Substituenten an der R⁴-Position ermöglichen eine optimale Kombination zwischen Zellpermeabilität und Affinität, die im Lichte des Standes der Technik nicht zu erwarten war.

In weiteren Ausführungsformen der Erfindung ist die erfindungsgemäße Verbindung durch die Formeln III, IV, V, VI, VII oder VIII gekennzeichnet:

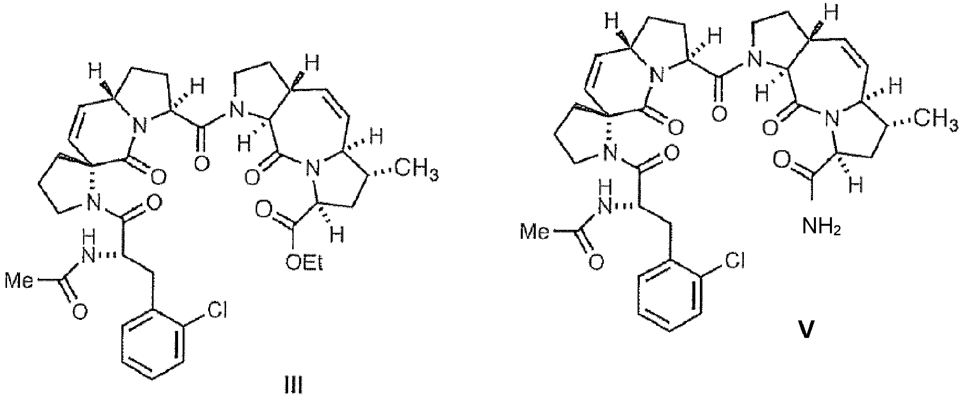

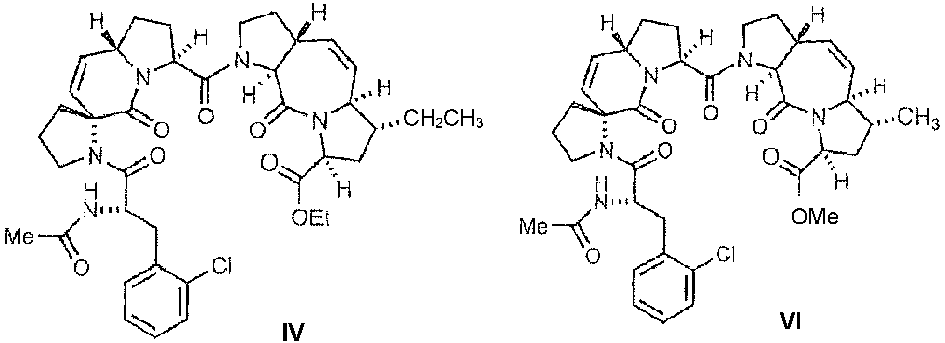

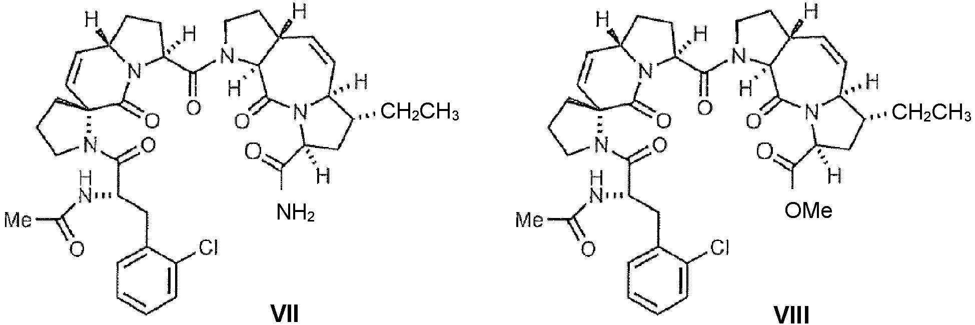

Die Erfindung betrifft eine Verbindung als Ligand für eine Ena/VASP-EVH1-Domäne. Die EVH1-Domäne besteht aus etwa 115 Aminosäuren und kommt in einer Vielzahl signalgebender Multidomänenproteine vor. Neben einigen weiteren gehört dazu auch die Familie der Ena/VASP-Proteine, die als molekulare Adaptoren wirken und die Actindynamik des Cytoskeletts modulieren. Man unterteilt die EVH1-Domänen nach ihrer Ligandenpräferenz in drei Klassen. Die erste Klasse erkennt insbesondere spezifisch ein [F/W/L]PPPP-Kernmotiv, das sich in fokalen Adhäsionsproteinen wie zum Beispiel Vinculin, Zyxin oder RIAM, in Lamellipodin, einem wichtigen Protein des Leitsaums, in einigen Proteinen des WAVE-Komplexes, wie auch im ActA-Protein des intrazellulären Bakteriums *Listeria monocytogenes* wiederfindet. Ena/VASP Proteine werden außerdem in Filopodien und Invadopodien nachgewiesen, wo sie mit ihrer EVH1-Domäne mit Forminen wechselwirken.

In einem weiteren Aspekt betrifft die Erfindung die Verwendung der erfindungsgemäßen Verbindungen als Ligand für eine Domäne ausgewählt aus der Gruppe umfassend Src-Homologie-3-Domänen, WW-Domänen, GYF-Domänen, UEV-Domänen und/oder Profilin.

Die Verbindung der vorliegenden Erfindung ist in einer Ausführungsform dadurch gekennzeichnet, dass die Verbindung eine Ena/VASP-EVH1 -vermittelte Protein-Protein-Wechselwirkung inhibiert. Demgemäß betrifft die Erfindung auch die Verwendung der neuen Strukturmimetika der Polyprolinll-Helix als überraschend gute Inhibitoren von Protein-Protein-Wechselwirkungen, bei denen insbesondere EVH1-Domänen, SH3-Domänen, WW-Domänen, GYF-Domänen, UEV-Domänen und Profilin beteiligt sind. Proteine, die diese Domänen enthalten, wie z. B. VASP (EVH1-Domäne) oder YAP (WW) spielen u. a. bei der Regulation der Zellmotilität (insbesondere VASP) und der Zellproliferation (insbesondere YAP) eine bedeutsame Rolle. So ist beispielsweise VASP in hochinvasiven Brustkrebszellen stark überexprimiert.

Die Verbindung der vorliegenden Erfindung ist in einer Ausführungsform dadurch gekennzeichnet, dass die Verbindung eine Dissoziationskonstante (Kd) im Komplex mit einer Ena/VASP-EVH1-Domäne von ≤ 20 µM, bevorzugt ≤ 2 µM, weiterhin bevorzugt ≤ 500 nM aufweist. Die besonders niedrigen Dissoziationskonstanten stellen ein überraschendes Ergebnis dar. Es war vorher weder bekannt noch im Stand der Technik erwähnt, dass Polyprolin-Mimetika solche Bindungseigenschaften an EVH1-Domänen aufweisen können.

In einem weiteren Aspekt betrifft die Erfindung eine pharmazeutische Zusammensetzung umfassend eine oder mehrere erfindungsgemäße Verbindungen, bevorzugt mit einem pharmazeutisch annehmbaren Träger. Bevorzugte pharmazeutische Träger sind beispielsweise Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

Die Erfindung betrifft in einem bevorzugten Aspekt die Verwendung der genannten Verbindungen als pharmazeutische Wirkstoffe. Die Verwendung als pharmazeutischer Wirkstoff betrifft die Verwendung für chirurgische, therapeutische oder diagnostische Verfahren. Die Erfindung betrifft in einem weiteren Aspekt ein pharmazeutisches Mittel, das die erfindungsgemäßen Verbindungen gegebenenfalls zusammen mit einem pharmazeutisch verträglichen Träger umfasst.

In einer bevorzugten Ausführungsform der Erfindung werden die Verbindungen als Poly-Prolin-Mimetika eingesetzt. Vorteilhafterweise findet man prolinreiche Aminosäurensequenzen insbesondere in Peptiden, die an Signaltransduktionsvorgängen, insbesondere intrazellulären Signaltransduktionsvorgängen beteiligt sind. Im Sinne der Erfindung kann der Begriff der Mimetika auch als Analoga verstanden werden. Bevorzugt ist die Verwendung der erfindungsgemäßen Verbindungen zur Behandlung von Krankheiten, die mit einer Modifikation von intrazellulären Signaltransduktionsvorgängen assoziiert sind, die durch Poly-Prolin-Helix-Strukturen vermittelt werden, ausgewählt aus der Gruppe umfassend Tumorerkrankungen, bakterielle Infektionskrankheiten und/oder neurodegenerative Erkrankungen.

In einem weiteren Aspekt betrifft die Erfindung die Verwendung der erfindungsgemäßen Verbindungen als Medikament zur Behandlung von Tumorerkrankungen. In einer bevorzugten Ausführungsform der Erfindung ist die Tumorerkrankung Brustkrebs.

Die Verwendung einer erfindungsgemäßen Verbindung als Medikament zur Behandlung einer Tumorerkrankung wird vorzugsweise dadurch gekennzeichnet, dass die Verbindung die Metastasierung eines Tumors, bevorzugt durch Inhibition der Chemotaxis und/oder Motilität der Tumorzellen auf Basis einer Störung der Aktinfilamentsynthese, inhibiert.

Es war völlig überraschend, dass die erfindungsgemäßen Verbindungen eine besonders gute Wirksamkeit gegen die Chemotaxis und/oder Motilität von Tumorzellen zeigen würden. Die Hemmung der Chemotaxis und/oder Motilität von Tumorzellen spielt daher insbesondere eine Rolle bei der Behandlung und/oder Prävention von Tumormetastasen. Dies stellt einen unterschiedlichen technischen Effekt im Vergleich zur Hemmung der Zellproliferation dar.

Aktin ist ein Strukturprotein, das in allen eukaryotischen Zellen vorkommt. Es ist Bestandteil des Zytoskeletts und eines der fünf häufigsten Proteine in Eukaryoten. Aktin bildet aneinandergereiht als F-Aktin dynamische Aktinfilamente. Diese Mikrofilamente dienen der Stabilisierung der äußeren Zellform, der Ausbildung von Zellfortsätzen, intrazellulären Verlagerungen und gerichteten zellulären Bewegungen. Viele eukaryotische Zellen besitzen ein hohes Maß an Bewegungsfähigkeit, Zellmotilität oder auch Zellmigration genannt, um beispielsweise Eindringlinge im Körper unschädlich machen zu können (Zellen des Immunsystems), Wunden heilen zu können (z. B. Hautzellen) oder um allgemein Zellen zu bewegen (in der Entwicklung oder bei einzelligen Organismen wie z. B. Amöben). Die Zellmigration spielt ebenfalls eine wesentliche Rolle bei Krebsmetastasen. Diese Beweglichkeit beruht hauptsächlich auf zwei Prozessen: Der gerichteten Aktinpolymerisierung in die Bewegungsrichtung (geregelt durch eine Anzahl von Regulatoren die auf Signale von der Zellperipherie reagieren), und der Aktin-Myosin-Interaktion in Fibrillenbündeln (stress fibers), kontraktile Zugseile, die durch die Zelle verlaufen und formgebende Elemente mit der Unterlage verspannen.

Die von den erfindungsgemäßen Verbindungen hervorgerufene Störung der Aktinfilamentsynthese stellt ebenfalls einen neuen und vorteilhaften technischen Effekt dar. Durch eine Störung der Aktinfilamentsynthese können die erfindungsgemäßen Verbindungen die Motilität der Krebszellen inhibieren und somit die Krebsmetastasierung stark hemmen bzw. dieser vorbeugen.

Die bevorzugten Tumorerkrankungen sind ausgewählt aus der Gruppe umfassend Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudo;, das Choristom; das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in-situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchioloalveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom; Chordom; Craniopharyngiom; Dysgerminom; Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadeno-carcinom; Cystadenom; Granulosa-zelltumor; Gynadroblastom; Hidradenom; Inselzelltumor; Leydig-Zelltumor; Papillom; Sertoli-Zell-Tumor; Thekazell-tumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paragangliom; nichtchromaffines Paragangliom; Angiokeratom; angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des ürogenitaltrakts); Neuro-fibromatose und zervikale Plattenepitheldysplasie.

In einer weiter bevorzugten Ausführungsform ist die Krebserkrankung oder der Tumor ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarm-krebs, der Ovarialkarzinome, der Zervikalkarzinome, Lungen-krebs, Prostatakrebs, Nierenzellkarzinome und/oder Lebermetastasen.

Bei der Behandlung der genannten Krankheiten ist es besonders bevorzugt, das pharmazeutische Mittel, welches die erfindungsgemäßen Verbindungen umfasst, als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zuzubereiten und/oder anzuwenden.

Bevorzugt liegt das pharmazeutische Mittel, welches die erfindungsgemäßen Verbindungen umfasst, in einer Konzentration von 0,1 bis 99,5, bevorzugt von 0,5 bis 95,0, besonders bevorzugt von 20,0 bis 80,0 Gew.-% in einer Zubereitung vor.

Bevorzugt wird diese Zubereitung oral, subkutan, intravenös, intramuskulär, intraperitoneal und/oder topisch gesetzt.

Bevorzugt wird das pharmazeutische Mittel, das die erfindungsgemäßen Verbindungen enthält, in Gesamtmengen von 0,05 bis 500 mg pro kg, bevorzugt von 5 bis 100 mg pro kg Körpergewicht, je 24 Stunden eingesetzt.

Bevorzugt erfolgt die Verabreichung der erfindungsgemäßen Verbindung oder pharmazeutischen Zusammensetzung in den Patienten oral, über Injektion, topisch, vaginal, rektal und/oder nasal.

Die vorliegende Beschreibung offenbart auch einen Kit, der mindestens eine der erfindungsgemäßen Verbindungen und/oder eines der erfindungsgemäßen pharmazeutischen Mittel, gegebenenfalls mit einer Information zum Kombinieren der Inhalte des Kits, umfasst - z. B. einen Beipackzettel oder eine Internet-Adresse, die auf Homepages mit weiteren Informationen verweist etc. Die Information zur Handhabung des Kits kann beispielsweise ein Therapie-Schema für die o. g. Krankheiten, insbesondere für die bevorzugten Krankheiten umfassen. Die Information kann jedoch auch Angaben darüber umfassen, wie die erfindungsgemäßen Erzeugnisse innerhalb einer Diagnose der genannten Krankheiten einzusetzen sind. Der erfindungsgemäße Kit kann auch in der Grundlagenforschung verwendet werden.

Die vorliegende Beschreibung offenbart auch die Verwendung des Kits zur Prophylaxe und/oder Therapie von neurodegenerativen Erkrankungen, bakteriellen Infektionserkrankungen oder Tumorerkrankungen.

### FIGUREN

- **Fig. 1:**: Hemmung der Chemotaxis und Motilität hoch invasiver Tumorzellen in einem Matrigel-Migrationsassay.
- **Fig. 2:**: Zelltoxizität der Verbindung gemäß Formel III.
- **Fig. 3:**: Bindung der Verbindung gemäß Formel III an ena-EVH1.
- **Fig. 4:**: Cyclus der angewendeten Festphasensynthese zum Aufbau der Peptidliganden.
- **Fig. 5:**: Bindung von SFEFPPPPTEDEL an VASP-EVH1.
- **Fig. 6:**: Verlauf der Ligandenoptimierung bzgl. des Enthalpiegewinns ΔΔG; links: ΔG bestimmt mittels ITC, rechts: ΔG: bestimmt mittels ITC.
- **Fig. 7:**: Oben: Bindung von Ac-**Phe(2-Cl)-[ProM-2][ProM-1]**-OH (hinten) und Ac-**Phe(2-Cl)-[ProM-2][ProM-9]**-OH (vorn) an der Proteinoberfläche, unten: Aussrichtung der zusätzlichen Methylgruppe in die Bindungstasche.

### BEISPIELE

Im Folgenden soll die Erfindung anhand der Synthese der erfindungsgemäßen Verbindungen näher erläutert werden, ohne auf dieses beschränkt zu sein.

### Beispiel 1: Synthese der Verbindungen

### Synthese von Fmoc-ProM-1:

Das tricyclische Dipeptidmimetikum Fmoc-ProM-1 wurde als erster PPII-Scaffold synthetisiert und als Prolin-Prolin-Äquivalent erfolgreich in Peptidliganden eingebaut.

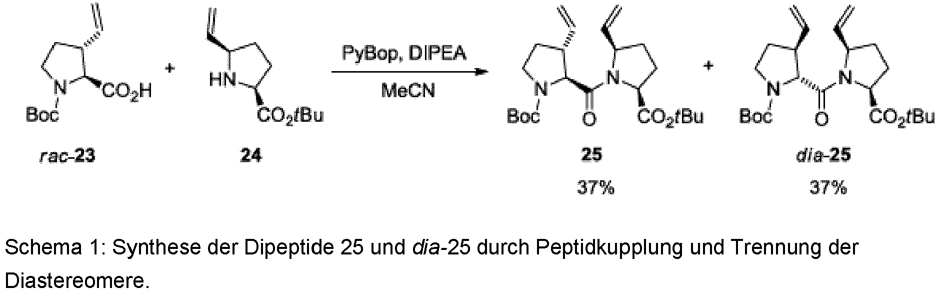

Im ersten Schritt wurde der racemische Säurebaustein rac-23 mit dem Amin 24 unter Einsatz von PyBop gekuppelt. Die dabei anfallenden Diastereomere 25 und dia-25 wurde nach säulenchromatographischer Trennung an Kiesegel jeweils in einer Ausbeute von 37% erhalten (Schema 1).

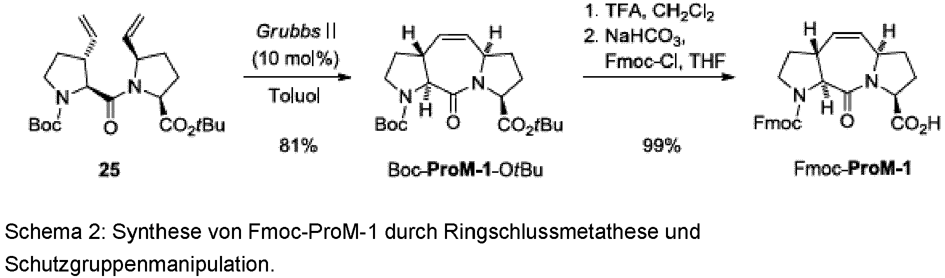

Im Anschluss wurde das Dipeptid 25 unter Verwendung des *Grubbs* II-Katalysators (10 mol%) in den Tricyclus Boc-ProM-1-OtBu überführt. Die Ringschlussmetathese wurde dabei unter Refluxieren in Toluol (120 °C) durchgeführt und lieferte das gewünschte Produkt nach säulenchromatographischer Reinigung in einer Ausbeute von 81%. Nach finaler Umwandlung von Boc-ProM-1-OtBu zu Fmoc-ProM-1 (TFA/NaHCO₃, Fmoc-Cl) wurde die Zielverbindung in 99% Ausbeute isoliert (Schema 2). Die Reinigung von Fmoc-ProM-1 wurde durch automatisierte Säulenchromatographie an einem *Reveleris*® Flash-Systems (MeOH/CH2Cl2; Gradient: 0 - 20%) ohne Zusatz von HOAc durchgeführt.

### Synthese von Fmoc-ProM-4

Hierzu wurde der racemische Baustein rac-67 nach Aktivierung (PyBop) mit dem Ostbaustein 24 gekuppelt. Das entsprechende Dipeptid 185 wurde als diastereomeres Gemisch (*dr* = 1:1, *cis*/*trans*) in einer Ausbeute von 82% erhalten. Eine säulenchromatographische Trennung der beiden Isomere gelang an dieser Stelle nicht (Schema 3).

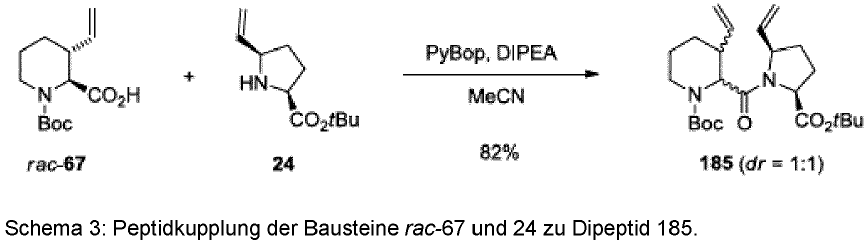

Nach Umsetzung von 185 mit *Grubbs* II-Katalysator (20 mol%) in refluxierendem CH2Cl2 wurde das cyclisierte Rohprodukt in Form eines diastereomeren Gemisches der beiden Isomere Boc-ProM-4-OtBu und *dia*-Boc-ProM-4-OtBu erhalten. Beide Diastereomere konnte auf dieser Stufe durch Säulenchromatographie an Kieselgel voneinander getrennt werden, sodass die gewünschte Verbindung Boc-ProM-4-OtBu in 42% Ausbeute neben *dia*-Boc-ProM-4-OtBu (ebenfalls in 42%) erhalten werden konnte (Schema 4).

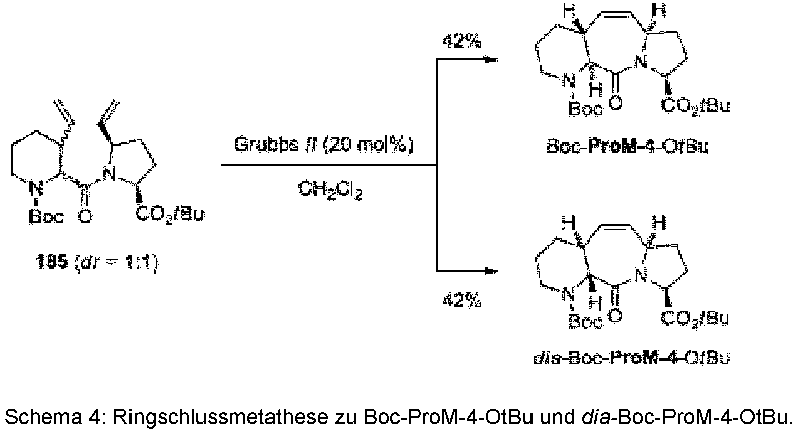

Durch vorsichtiges Entfernen des Lösungsmittels nach der chromatographischen Reinigung konnten beide Isomere in kristalliner Form erhalten werden. Die Qualität der Kristalle reichte zur Bestimmung der relativen Konfiguration durch Röntgenstrukturanalyse aus. Nach erfolgreichem Ringschluss und Trennung der Diastereomere sollte Verbindung Boc-ProM-4-OtBu im finalen Schritt durch globale Entschützung mit TFA und anschließender *N*-terminaler Fmoc-Schützung (NaHCO3, Fmoc-Cl) in das anvisierte Mimetikum Fmoc-ProM-4 überführt werden (Schema 4.90). Nach säulenchromatographischer Reinigung des Rohprodukts mittels eines *Reveleris*® Flash-Säulenchromatographie-Systems (MeOH/CH2Cl2; Gradient: 0 - 20%) konnte das gewünschte Mimetikum Fmoc-ProM-4 in einer Ausbeute von 80% isoliert werden.

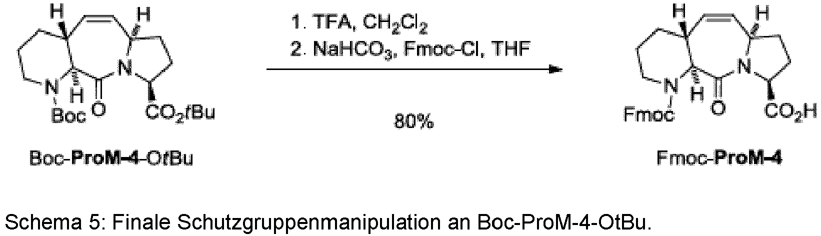

### Synthese von Fmoc-ProM-9:

Für die Kupplung zum Dipeptid wurde an dieser Stelle nicht der racemische Säurebaustein rac-23 sondern die enantiomerenreine Verbindung 23 verwendet, um nach Kupplung mit dem Aminbaustein 70 eine möglicherweise schwierige Trennung der resultierenden Diastereomere zu vermeiden. Aus dem Moc-geschützten Prolin 34, welches mittels chiraler, präparativer HPLC durch die *Bayer Pharma* AG *(HealthCare Pharmaceuticals)* in enantiomerenreiner Form zur Verfügung gestellt wurde, konnte durch Schutzgruppenmanipulation die benötigte *N*-Boc-Carbonsäure 23 in einer Ausbeute von 64% über 3 Stufen erhalten werden (Schema 6).

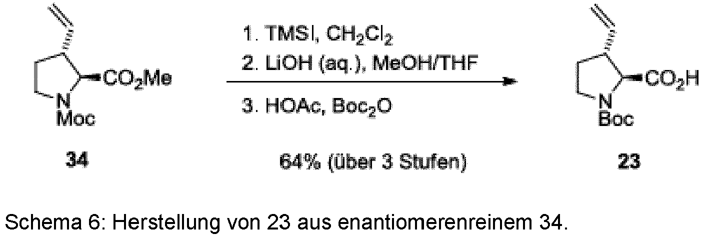

Nach der Synthese des enantiomerenreinen Westbausteins 23 wurde dieser unter Standardbedingungen mit dem Methylprolinderivat 70 gekuppelt. Nach Aktivierung mit PyBop konnte durch Reaktion bei Raumtemperatur und anschließender säulenchromatographischer Reinigung des Rohprodukts an Kieselgel das gewünschte Dipeptid 192 in einer Ausbeute von 87% isoliert werden (Schema 7).

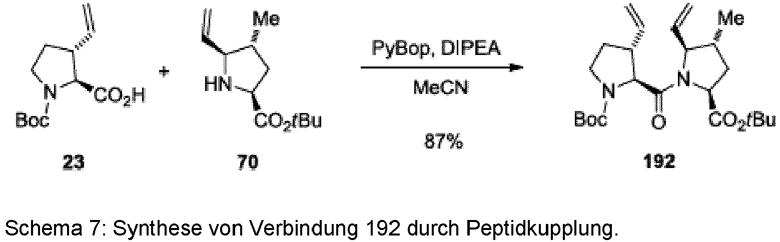

Die nachfolgende Ringschlussmetathese von 192 unter Bildung des tricyclischen Produkts Boc-ProM-9-OtBu wurde durch Einsatz von 10 mol% *Grubbs* II-Katalysator in refluxierendem CH₂Cl₂ durchgeführt. Ein vollständiger Umsatz konnte schon nach 8 h detektiert werden. Nach säulenchromatographischer Reinigung wurde das cyclisierte Produkt Boc-ProM-9-OtBu in 82% Ausbeute erhalten (Schema 8). Wie schon im Falle der Metathese von Boc-ProM-4-OtBu konnte nach der Säulenchromatographie, durch vorsichtiges Entfernen der Lösungsmittelreste, die Substanz in kristalliner Form erhalten und in ihrer Struktur röntgenanalytisch untersucht werden.

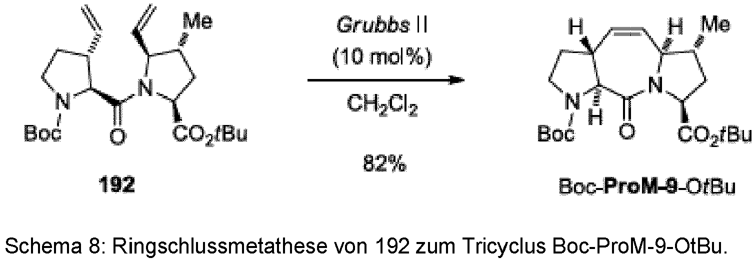

Im finalen Schritt der Synthese des Scaffolds Fmoc-ProM-9 bedurfte es der Schutzgruppenmanipulation am tricyclischen System Boc-ProM-9-OtBu. Nach bekannter Prozedur wurde die Verbindung global mit TFA entschützt und nachfolgend mit Fmoc-Cl im leicht basischen Milieu (NaHCO3, pH = 7-8) *N*-terminal geschützt. Nach automatisierter Reinigung (*Reveleris*® -System, MeOH/CH₂Cl₂; Gradient: 0 - 20%) konnte das gewünschte Mimetikum Fmoc-ProM-9 in einer Ausbeute von 92% isoliert werden (Schema 9).

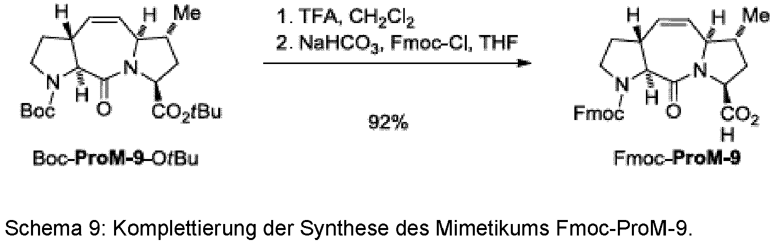

### Synthese von Fmoc-ProM-2:

Zum Aufbau der dipeptidischen Verbindung 46 wurden die beiden Bausteine 45 und *epi-24* mit HATU und DIPEA in NMP bei 85 °C in einer Ausbeute von 60% gekuppelt. In diesem Zusammenhang war auf eine hohe (trockene) Qualität des Kupplungsreagenzes zu achten. Zur Ringschlussmetathese des Dipeptids 46 wurden die beschriebenen Bedingungen (30 mol% *Grubbs* II-Katalysator in CH₂Cl₂, Refluxieren) zur Erhöhung der Ausbeute (von nur 55%) verändert. So konnte nach Reaktion in refluxierendem Toluol (110-120 °C) der gewünschte Tricyclus Boc-ProM-2-OtBu in einer Ausbeute von 68% erhalten werden. Im finalen Schritt der Synthese von Fmoc-ProM-2 wurde das Metatheseprodukt zunächst global entschützt (TFA) und unter basischen Bedingungen (NaHCO3, pH = 7-8) *N*-terminal Fmoc-geschützt. Nach säulenchromatographischer Reinigung des Rohprodukts mittels eines *Reveleris*® Flash-Säulenchromatographie-Systems (MeOH/CH2Cl2; Gradient: 0 - 20%) konnte das anvisierte Mimetikum Fmoc-ProM-2 in einer Ausbeute von 90% isoliert werden. In Schema 4.98 ist die Synthesesequenz zum Aufbau von Fmoc-ProM-2 ausgehend von den Prolinbausteinen 45 und *epi-24* nochmals dargestellt.

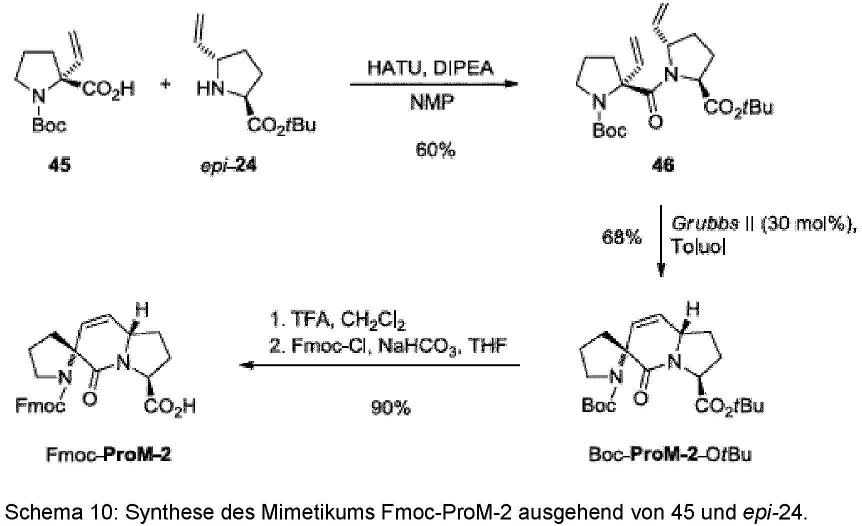

### Synthese von Fmoc-ProM-13:

Die Synthese des Scaffolds Fmoc-ProM-13 wurde auf Basis des folgenden Schemas (Schema 11) durchgeführt: 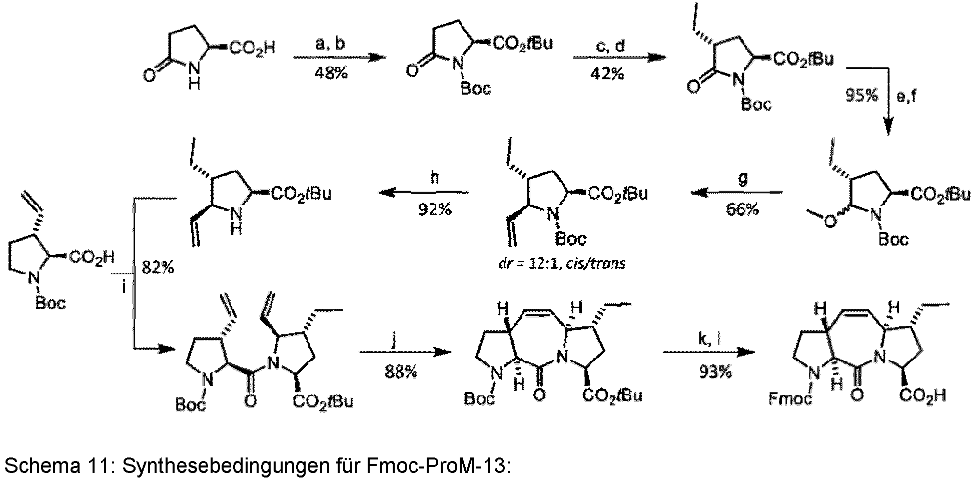 (a) HClO4, tBuOAc, RT, 65 h; (b) Boc2O, DMAP, MeCN, RT, 18 h; (c) LiHMDS, EtOTf, TPPA, THF, - 78°C, 6 h; (d) TBAF, THF, 80°C, 1.5 h; (e) DIBAL-H, THF, -78°C, 2.5 h; (f) PPTS, MeOH, RT, 16 h; (g) VinylMgBr, AICI3, BF3.OEt2, -40 °C, 6 h; (h) TMSOTf, CH2Cl2, 0 °C, 20 min; (i) PyBOP, DIPEA, MeCN, RT, 23h; (j) Grubbs II, CH2Cl2, RT, 8 h; (k) TFA, CH2Cl2, 0 °C, 1 h; (I) NaHCO3, FmocCl, THF, RT, 16 h.

Fmoc-ProM-13 weist die folgende Struktur auf:

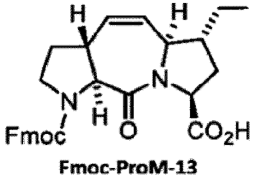

### Festphasenpeptidsynthese und Liganden-Funktionalisierung:

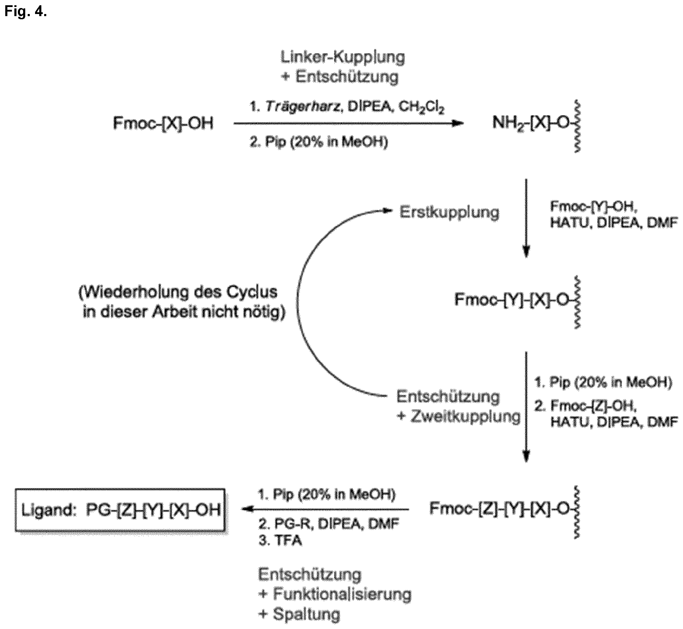

Als Verfahren zur Herstellung der Verbindungen diente die Festphasenpeptidsynthese (SPPS, engl.: *solid phase peptide synthesis).* Der, von *Merrifield* 1963 entwickelte, kontrollierte Aufbau eines Polypeptids an einem festen funktionalisierten Polystyrolharz erlaubt dabei die schnelle, effektive und gegebenenfalls automatisierte Synthese von Peptiden unter milden Bedingungen. Zur schrittweisen Verdeutlichung des Kupplungscyclus ist der entsprechende Ablauf in Fig. 4 exemplarisch dargestellt. So wird zunächst entgegen der "natürlichen" Syntheserichtung die erste Aminosäure über den C-Terminus an das Polystyrolharz gekuppelt. Nach *N*-terminaler Entschützung (üblicherweise Boc-, oder Fmoc-Schutzgruppenstrategien) kann anschließend eine weitere Aminosäure durch Aktivierung mit einem Kupplungsreagenz an die festphasengebundene Peptidsequenz angebracht werden. Dieser Cyclus kann belieb oft wiederholt werden, wobei durch Waschen des Trägerharzes überschüssige Reagenzien entfernt werden. Zur Isolierung des freien Peptids wird am Ende die Abspaltung vom Harz durchgeführt.

Unter der Anwendung einer Fmoc-Schutzgruppenstrategie wurde die Synthese von Ac-Pro-Pro-Pro-OH als Test-Verbindung (sowie der weiteren Verbindungen) in einem Reaktor unter vorsichtigem Rühren durchgeführt. Dieser bestand aus einer PET-Spritzenhülse, verbunden mit einer Pumpe zum Absaugen von Lösungsmittelresten bzw. überschüssigen Reagenzien, und konnte hierzu beliebig über eine Klammer geöffnet und geschlossen werden. Als Linker wurden Chlor(2'-chlor)trityl-Polystyrolharz-Kugeln (beads) verwendet. Als Kupplungsreagenz diente HATU in DMF. Eine 20%ige Piperidin-Lösung (DMF) wurde zur Fmoc-Entschützung eingesetzt. Detailliertere Angaben zu den verwendeten Reaktionsbedingungen, sowie den Waschprozeduren sind dem Experimentalteil zu entnehmen. Sämtliche Liganden wurden am Ende durch Zugabe von TFA vom Harz abgespalten und durch anschließende Lyophilisierung und Reinigung durch HPLC für die späteren biologischen Tests vorbereitet. Auf die synthetisierten Liganden und Funktionalisierungen (NBD-Labeling, Veresterung) soll im Folgenden eingegangen werden. So konnte nach der beschriebenen Prozedur ausgehend von Fmoc geschütztem L-Prolin (193) nach doppelter Peptidkupplung mit 189 und anschließender Acetylierung mit Ac₂O das gewünschte Triprolinderivat Ac-Pro-Pro-Pro-OH an der festen Phase synthetisiert werden (Fig. 4). Die erfolgreiche Synthese der Substanz konnte jedoch durch LCMS-Analytik bestätigt werden.

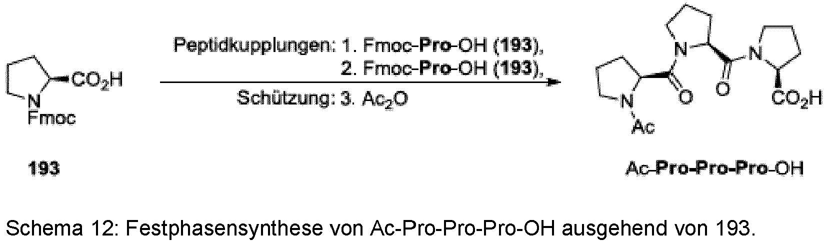

Zur C-terminalen Veresterung des Triprolins Ac-Pro-Pro-Pro-OH wurde die Substanz unter klassischen *Steglich*-Bedingungen (DCC, DMAP) mit EtOH bei Raumtemperatur zur Reaktion gebracht. Nach einer Reaktionszeit von 23 h konnte der gewünschte Ethylester Ac-Pro-Pro-Pro-OEt als Rohprodukt zur Reinigung mittels HPLC übergeben werden. Die erfolgreiche Synthese der Substanz konnte wiederum durch LCMS-Analytik bestätigt werden.

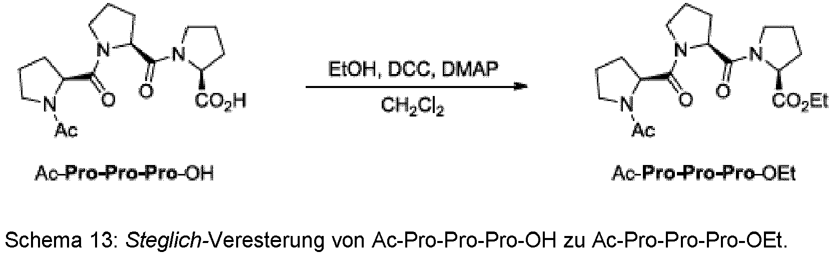

Nach der Entwicklung einer erfolgreichen Methodik zur Veresterung von prolinreichen Liganden des Typs Ac-Pro-Pro-Pro-OEt findet diese Prozedur für die Synthese des Ethylester-Liganden Ac-Phe(2-Cl)-[ProM-2]-[ProM-1]-OEt Anwendung. Hierzu bedurfte es zunächst dem Aufbau des Systems Ac-Phe(2-Cl)-[ProM-2]-[ProM-1]-OH mittels Festphasenpeptidsynthese, wobei die schon in den vorherigen Kapiteln diskutierten Fmoc-Mimetika Fmoc-ProM-1 und Fmoc-ProM-2 zum Einsatz kamen. In dieser Reihenfolge wurden beide Dipeptidanaloga an die feste Phase gebracht, im letzten Schritt mit L-2-Chlor-Phenylalanin gekuppelt und mit Ac₂O *N*-terminal acetyliert (Schema 14).

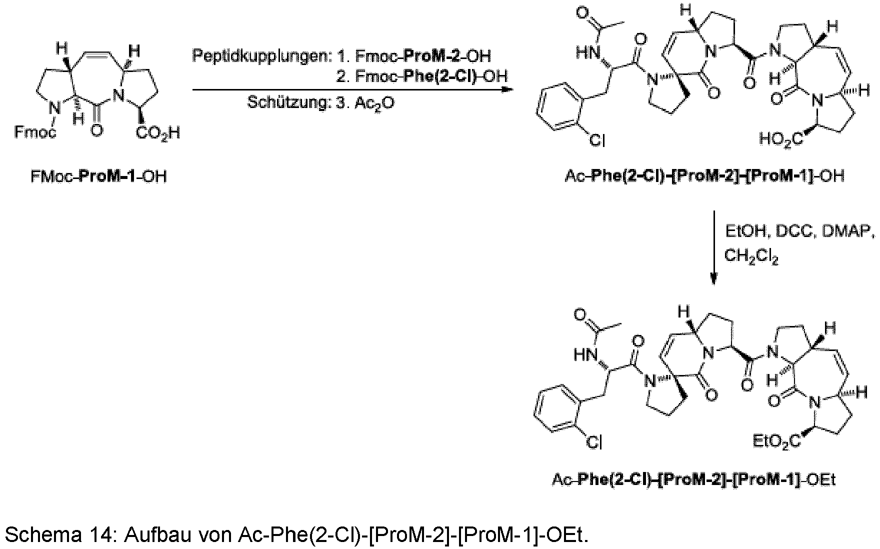

Im Folgeschritt konnte der Ligand Ac-Phe(2-Cl)-[ProM-2]-[ProM-1]-OH erfolgreich durch Anwendung der *Steglich*-Veresterung in sein Ethylesterderivat Ac-Phe(2-Cl)-[ProM-2]-[ProM-1]-OEt überführt werden (Nachweis mittels LCMS-Analytik). Nach identischem Synthesemuster wurde auch der NBDgelabelte Ligand Ac-Phe(2-Cl)-[ProM-2]-[ProM-1]-OEt hergestellt (Schema 15), sodass nun beide Formen (acetyliert und NBD-gelabelt) als Ethylester für biologische Tests zur Verfügung standen.

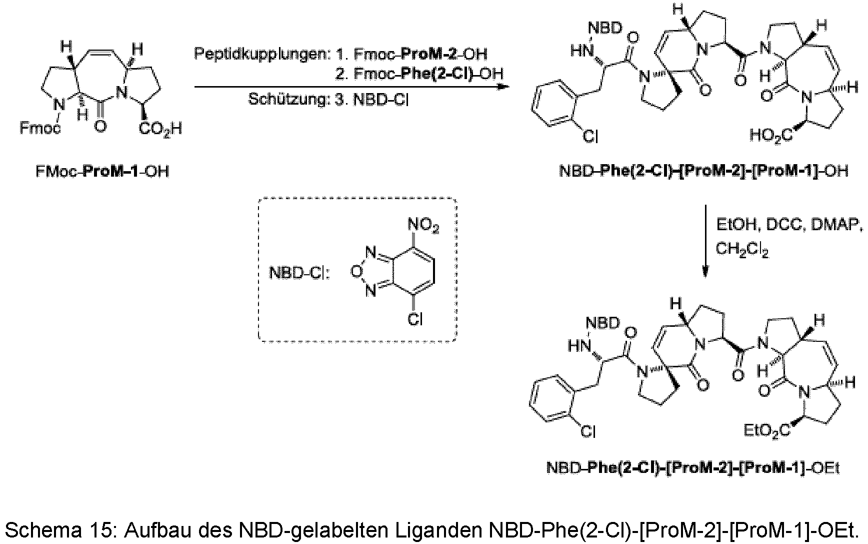

### Versuche zur Festphasensynthese der Peptidliganden:

Allgemeine Versuchsvorschrift (A) zur Festphasensynthese der Liganden:

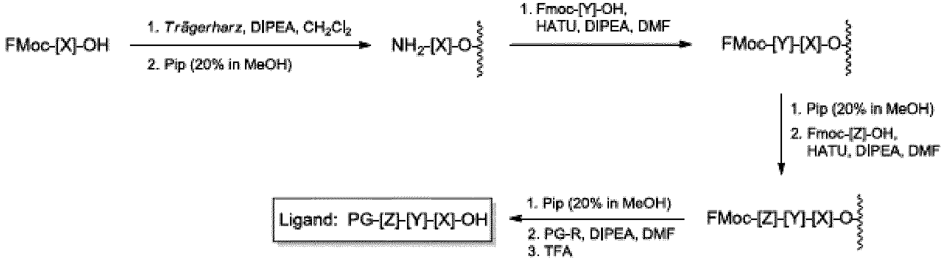

Harzkupplung: In einer offenen Spritzenhülse, verbunden mit einem Absaugmechanismus, wurde Chlor-(2'-chlor)trityl-Polystyrolharz (1 g / 1 mmol Säure, Kapazität = 1.19 mmol/g) in trockenem CH2Cl2 suspendiert und nach Zugabe von FMoc-[X]-OH (1.0 Äq.) und DIPEA (2.0 Äq.) bei RT für 2 h vorsichtig gerührt. Im Anschluss wurde das Gemisch mit MeOH/DIPEA (9:1) neutralisiert, für 15 min gerührt und nach Entfernen der Lösung mit DMF (3 x 10 ml) gewaschen.

Peptidkupplung(en): Nach Entfernen des DMF wurde der Rückstand zweimal nacheinander in einer Piperidin-Lösung (10 ml, 20%ig in MeOH) bei RT für 15 min gerührt. Nach Entfernen der Lösung wurde mit CH2Cl2 (4 x 10 ml) nachgewaschen. Nach Entfernen des CH2Cl2 wurde eine Lösung aus Fmoc-[Y/Z]-OH (1.2 - 4.0 Äq.), HATU (2.0 - 4.0 Äq.) und DIPEA (4.0 - 8.0 Äq.) in DMF voraktiviert (3 min Ultraschallbad) und bei RT mit dem Trägerharz für 25 min gerührt. Im Anschluss wurde das LM entfernt und der Rückstand mit DMF und CH2CI2 (jeweils 2 x 10 ml) gewaschen. Nach Entfernen der Waschlösung und eventuellem Kaisertest wurde das Harz mit einem Gemisch aus Ac2O, DIPEA und DMF (10 ml, 1:2:7) bei RT für 10 min gerührt und mit DMF (3 x 10 ml) nachgewaschen.

Die Prozedur der Peptidkupplung konnte beliebig oft durchgeführt werden. Für die diskutierten Liganden wurde die Prozedur zweifach durchgeführt.

Schützung und Harzabspaltung: Nach Entfernen der Waschlösung wurde der Rückstand zweimal nacheinander in einer Piperidin-Lösung (10 ml, 20%ig in MeOH) bei RT für 15 min gerührt. Nach Entfernen der Lösung wurde mit CH2CI2 (4 x 10 ml) nachgewaschen und die Waschlösung entfernt. Im Anschluss wurde der Rückstand entweder zur Ac-Schützung mit einem Gemisch aus Ac2O, DIPEA und DMF (10 ml, 1:2:7) bei RT für 10 min gerührt, oder Experimenteller Teil 270 zur NBD-Schützung mit einer Lösung aus NBD-CI (3.0 Äq.) und DIPEA (3.0 Äq.) in 2 ml DMF bei RT für 18 h geschüttelt. Das LM wurde entfernt, der Rückstand mit DMF (3 x 10 ml) gewaschen und die Waschphase entfernt. Zur finalen Harzabspaltung wurde das trockene Harz mit (2 - 10 ml) TFA bei RT für 2 h gerührt. Nach Entfernen der TFA wurde das Trägerharz mit TFA und CH2Cl2 (jeweils 3 x 10 ml) gewaschen. Die Waschlösungen wurden vereint und bis zur Trockne eingedampft. Das Produkt wurde nach Zugabe von Et2O (50 ml) unter Kühlung (Kühlschrank) gefällt, abgesaugt und mit Et2O (20 ml) gewaschen. Trocknen durch Lyophilisierung (MeCN/H2O; 10 - 20 ml) lieferte den entsprechenden Peptidliganden.

### Allgemeine Versuchsvorschrift (B) zur Steglich-Veresterung der Liaanden:

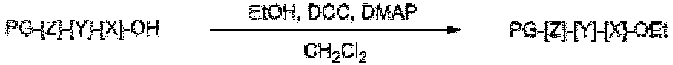

Unter Argonschutzgasatmosphäre wurde der entsprechende Ligand (1.0 Äq.) in trockenem CH2Cl2 (2 ml) gelöst und nach Zugabe von trockenem EtOH (5.0 - 10.0 Äq.), DCC (1.0 Äq.) und DMAP (0.1 - 0.2 Äq.) bei RT für 20 h gerührt. Im Anschluss wurde das LM unter vermindertem Druck entfernt, der Rückstand in CH2Cl2 (5 ml) aufgenommen, über eine Glasfritte filtriert (Porengröße = 4 Å) und mit CH₂Cl₂ (3 × 5 ml) nachgewaschen. Eindampfen des Filtrats unter vermindertem Druck lieferte den gewünschten Ester als Rohprodukt.

### Synthese von Ac-Phe(2-Cl)-[ProM-2]-[ProM-1]-OH nach Versuchsvorschrift (A):

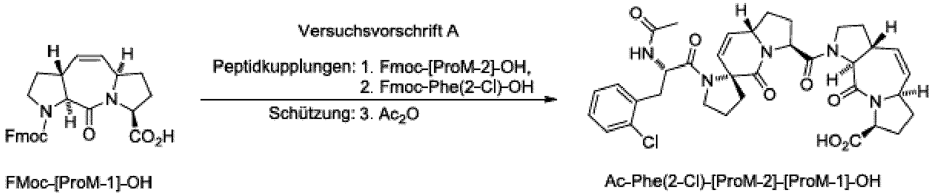

Unter Anwendung der Allgemeinen Versuchsvorschrift A (8.2.7.1) wurde Fmoc-[ProM-1]-OH (0.136 g, 0.297 mmol) mit Chlor(2'-chlor)trityl-Polystyrolharz (0.250 g) durch Zugabe von DIPEA (101 µl, 0.594 mmol) in CH₂Cl₂ (1.7 ml) gekuppelt. Zur Peptidkupplung wurde zuerst eine Lösung aus Fmoc-[ProM-2]-OH (0.164 g, 0.357 mmol), HATU (0.136 g, 0.357 mmol) und DIPEA (124 µl, 0.714 mmol) in DMF (1.5 ml) und dann eine Lösung aus Fmoc-Phe(2-Cl)-OH (0.251 g, 0.595 mmol), HATU (0.226 g, 0.595 mmol) und DIPEA (340 µl, 1.200 mmol) in DMF (1.5 ml) verwendet. Im Anschluss wurde das freie Amin mit einem Gemisch aus Ac₂O, DIPEA und DMF (10 ml, 1:2:7) acetyliert und mit 5 ml TFA vom Harz gespalten. Es konnten 0.075 g des Rohpeptids als weißer Feststoff erhalten werden. Das Rohprodukt wurde im Rahmen der Arbeit nicht weiter aufgereinigt. (Anschließende Aufreinigung mittels präparativer HPLC). LCMS-TOF-MS (ESI): berechnet für: [M+H]+ 678.270; gefunden: 678.268.

### Synthese von NBD-Phe(2-Cl)-[ProM-2]-[ProM-1]-OH nach Versuchsvorschrift (A):

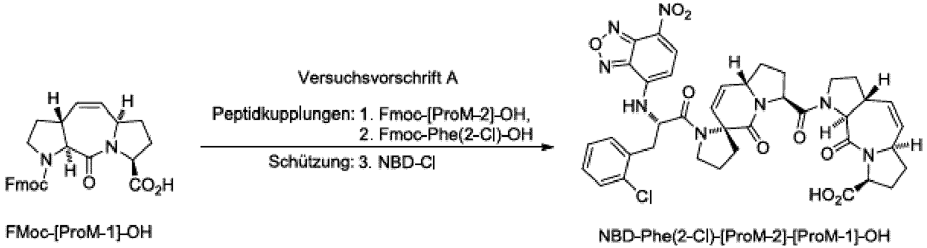

Unter Anwendung der Allgemeinen Versuchsvorschrift A (8.2.7.1) wurde Fmoc-[ProM-1]-OH (0.075 g, 0.164 mmol) mit Chlor(2'-chlor)trityl-Polystyrolharz (0.140 g) durch Zugabe von DIPEA (60 µl, 0.328 mmol) in CH₂Cl₂ (1.5 ml) gekuppelt. Zur Peptidkupplung wurde zuerst eine Lösung aus Fmoc-[ProM-2]-OH (0.090 g, 0.196 mmol), HATU (0.075 g, 0.196 mmol) und DIPEA (150 µl, 0.820 mmol) in DMF (1.5 ml) und dann eine Lösung aus Fmoc-Phe(2-Cl)-OH (0.138 g, 0.328 mmol), HATU (0.125 g, 0.328 mmol) und DIPEA (120 µl, 0.656 mmol) in DMF (1.5 ml) verwendet. Im Anschluss wurde das freie Amin mit NBD-CI (0.098 g, 0.492 mmol) und DIPEA (90 µl, 0.492 mmol) in DMF (10 ml) geschützt mit 3 ml TFA vom Harz gespalten. Es konnten 0.061 g des Rohpeptids als weißer Feststoff erhalten werden. Das Rohprodukt wurde im Rahmen der Arbeit nicht weiter aufgereinigt. (Anschließende Aufreinigung mittels präparativer HPLC). LCMS-TOF-MS (ESI): berechnet für:
[M+H]+ 799.261; gefunden: 799.251.

### Synthese von Ac-Phe(2-Cl)-[ProM-2]-[ProM-1]-OEt nach Versuchsvorschrift (B):

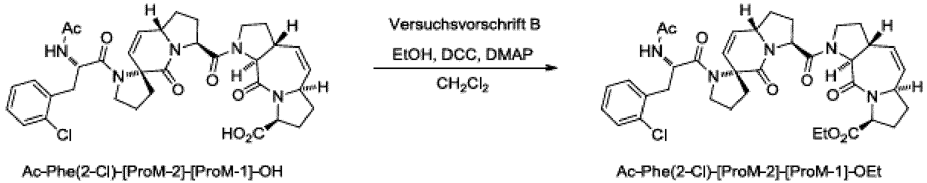

Unter Anwendung der Allgemeinen Versuchsvorschrift B (8.2.7.2) wurde Ac-Phe(2-Cl)-[ProM-2]-[ProM-1]-OH (0.035 g, 0.052 mmol) mit EtOH (100 µl), DCC (0.011 g, 0.052 mmol) und DMAP (0.001 g, 0.010 mmol) in CH₂Cl₂ (2 ml) verestert. Nach Aufarbeitung konnten 0.064 g des Rohprodukts als weißer Feststoff erhalten werden. Das Rohprodukt wurde im Rahmen der Arbeit nicht weiter aufgereinigt. (Anschließende Aufreinigung mittels präparativer HPLC). LCMS-TOF-MS (ESI): berechnet für: [M+H]+ 706.301; gefunden: 706.292. HRMS (ESI): berechnet für: [M+H]+ 706.301; gefunden: 706.302; berechnet für: [M+Na]+ 728.283; gefunden: 728.284. 8.2.7.8 Synthese von NBD-Phe(2-Cl)-[ProM-2]-[ProM-1]-OEt nach Versuchsvorschrift (B)

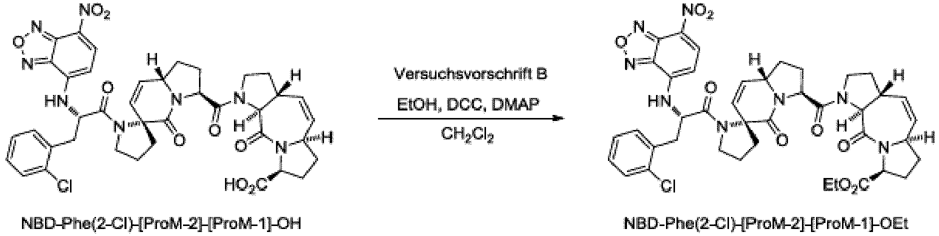

Unter Anwendung der Allgemeinen Versuchsvorschrift B (8.2.7.2) wurde NBD-Phe(2-Cl)-[ProM-2]-[ProM-1]-OH (0.061 g, 0.076 mmol) mit EtOH (200 µl), DCC (0.016 g, 0.076 mmol) und DMAP (0.001 (5) g, 0.015 mmol) in CH₂Cl₂ (2 ml) verestert. Nach Aufarbeitung konnten 0.091 g des Rohprodukts als weißer Feststoff erhalten werden. Das Rohprodukt wurde im Rahmen der Arbeit nicht weiter aufgereinigt. (Anschließende Aufreinigung mittels präparativer HPLC). LCMS-TOF-MS (ESI): berechnet für: [M+H]+ 827.292; gefunden: 827.294.

### Synthese in einer Lösung:

Als eine Alternative zu der Festphasenkopplung können die erfindungsgemäßen Verbindungen durch Synthese in einer Lösung hergestellt werden.

Folgendes Schema wird für den Schutz und die Aktivierung der Verbindungen verwendet:

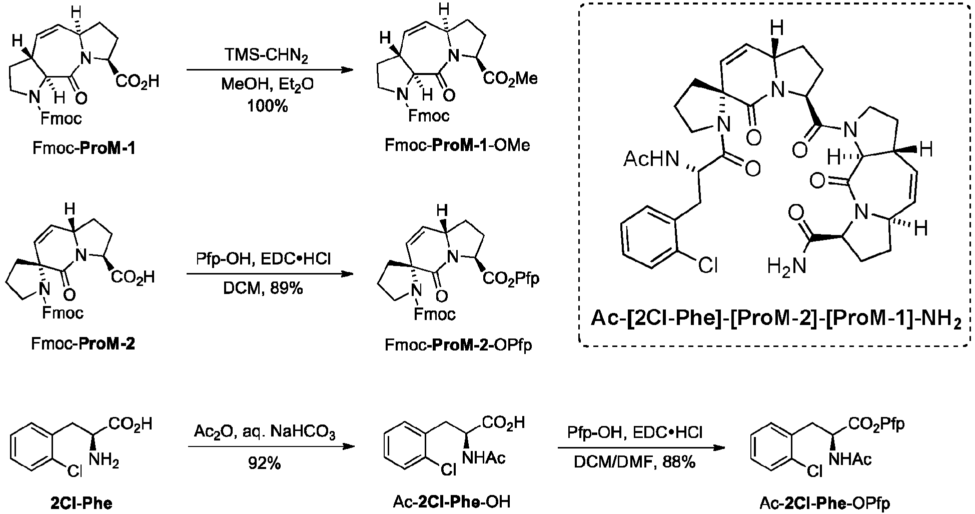

Anschließend können die verschiedenen Scaffolds auf Basis des folgenden Schemas gekoppelt werden:

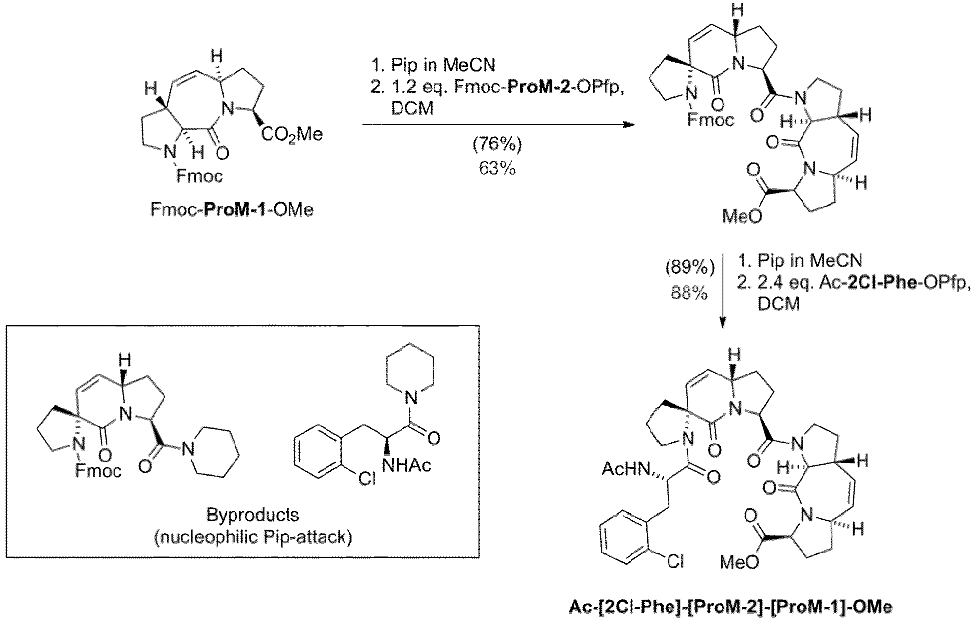

Die dadurch produzierten Verbindungen können durch eine direkte Amidierung modifiziert werden:

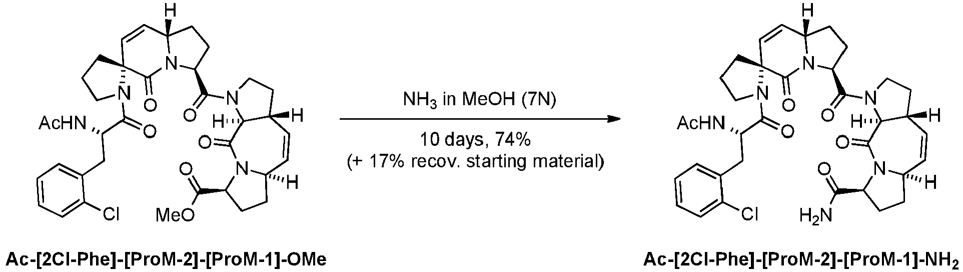

### Beispiel 2: Biologische Untersuchungen

Im Folgenden soll die Erfindung anhand der biologischen Eigenschaften der erfindungsgemäßen Verbindungen näher erläutert werden, ohne auf diese beschränkt zu sein.

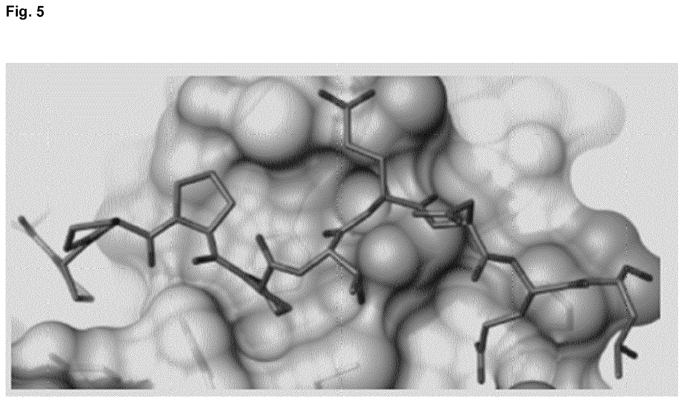

Als "Ausgangsligand" für Bindungsstudien wurde zunächst die "wild typ"-Sequenz Ac-SFEFPPPPTEDEL-NH2 (ein Ausschnitt aus dem ActA Protein des intrazellulären Bakteriums *Listeria monocytogenes,* Fig. 5) auf das FPPPP-Kernmotiv, welches spezifisch durch EVH1-Domänen erkannt wird, reduziert. Dadurch lag die Masse des resultierenden Liganden Ac-FPPPP-OH erstmalig im niedermolekularen Bereich (< 500 bzw. < 900 Da, je nach Definition), im Sinne eines "small molecules" zur Beeinflussung von Protein-Protein-Interaktionen.

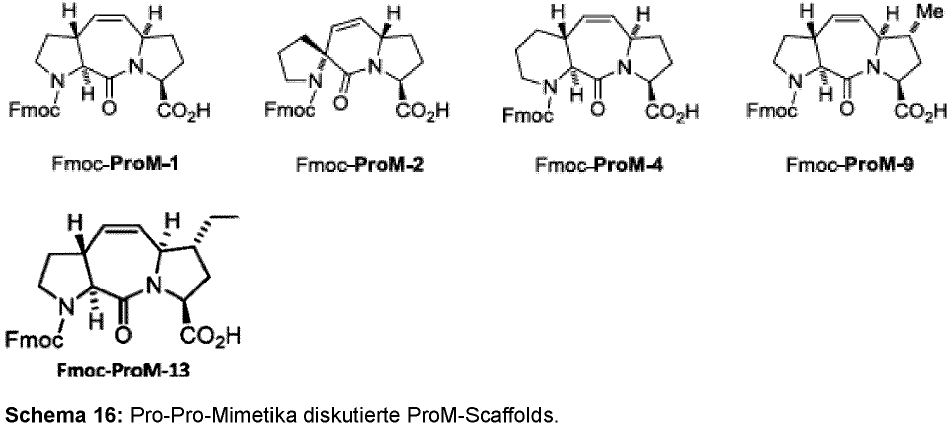

Die Substitution der Diprolineinheiten des FPPPP-Motivs mit den oben dargestellten Fmoc-ProMs wurde durchgeführt. Im Anschluss wurden die Bindungsaffinitäten der Liganden zur Domäne mittels isothermaler Titrationscalometrie (ITC) anhand der Dissoziationskonstante (Kd) bzw. der freien Enthalpie (ΔG) der Protein-Liganden-Komplexe bestimmt. Zusätzlich wurden die Ergebnisse der Messungen durch Fluoreszenztitration (FT) kontrolliert. Dabei drückt sich eine stärkere Bindung des jeweiligen Liganden durch einen kleineren Wert für Kd bzw. ein stärker negativen Wert für ΔG aus. Der Zusammenhang zwischen der Dissoziationskonstante (Kd) und der freien Enthalpie (ΔG) ist mit den nachfolgenden Gleichungen verdeutlicht:

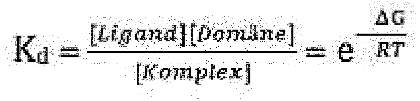

Die gemessenen Bindungsaffinitäten der synthetischen Liganden sind in Tabelle 1 aufgeführt.

**Tabelle 1: Bindungsaffinitäten der verschiedenen Liganden bezüglich der EVH-1-Domäne.**

| **Ligand** | **ITC** | | **FT** | |
|---|---|---|---|---|
| | Kd[µM] | ΔG[kJ/mol] | Kd[µM] | ΔG[kJ/mol] |
| Ac- SFEFPPPPTEDEL-NH2 | 20 | --- | --- | --- |
| Ac-FPPPP-OH | 1300 | -16.5 | 780 | -17.7 |
| Ac-Phe(2-Cl)-PPPP-OH | 18 | -27.0 | 13 | -27.8 |
| Ac-Phe(2-Cl)-PP[ProM-1]-OH | 14.1 | -27.7 | 3.2 | -31.4 |
| Ac-Phe(2-Cl)-PP[ProM-9]-OH | 8.0 | -29.1 | 0.4 | -36.7 |
| Ac-Phe(2-Cl)-[ProM-2][ProM-1]-OH | 3.8 | -31.0 | 2.7 | -31.8 |
| Ac-Phe(2-Cl)-[ProM-2][ProM-4]-OH | 9.7 | -28.6 | 1.6 | -33.1 |
| Ac-Phe(2-Cl)-[ProM-2][ProM-9]-OH | 0.69 | -35.2 | 0.28 | -37.4 |

Die Verkürzung des Ausgangsliganden Ac-SFEFPPPPTEDEL-NH2 auf das zentrale FPPPP-Motiv hatte zunächst eine deutlich schlechtere Bindungsaffinität des resultierenden, niedermolekularen Liganden Ac-FPPPP-OH zur Folge (Kd[ITC] = 20 µM auf 1300 µM). Ein Austausch des endständigen Phenylalanins (F) durch synthetisches 2-Chlor-Phenylalanin (Phe(2-Cl)) konnte die schlechtere Bindung des verkürzten Liganden kompensieren.

In weiteren Untersuchungen wurde das PPPP-Motiv nun durch die dipeptidischen Scaffolds ProM-1, ProM-2, ProM-4, ProM-9 und ProM-13 substituiert. Eine erneute Verbesserung der Bindungsaffinität konnte für den Austausch einer einzigen Pro-Pro-Dipeptideinheit durch ProM-1, oder ProM-9 beobachtet werden, wobei eine Substitution des vorderen, oder mittleren Pro-Pro-Einheit zu einem Verlust der Affinität führte. Zum Erhalt des ersten vollsynthetischen ProM-tragenden Peptidliganden wurden die beiden verbliebenen Proline durch die Struktur ProM-2 ersetzt. So konnten die Modelingstudien durch biologische Experiment bestätigt werden.

Es zeigte sich durch ITC-Messungen, dass der Ligand Ac-Phe(2-Cl)-[ProM-2][ProM-1]-OH im Vergleich zu Ac-Phe(2-Cl)-PP[ProM-1]-OH einen vierfach geringeren Kd-Wert besitzt. Eine Fluoreszenztitration bestätigte die gleiche Tendenz, jedoch nur mit einer Verbesserung der Bindungsaffinität um den Faktor 1.2. Dieser Wert konnte durch den Austausch von ProM-1 mit dem ringerweiterten Scaffold ProM-4 (Ligand: Ac-Phe(2-Cl)-[ProM-2][ProM-4]-OH) weiter optimiert werden (Halbierung von Kd). Im Gegensatz hierzu deuteten die entsprechenden ITC-Messungen auf schlechtere Bindungsaffinitäten hin, sodass der ProM-1-substituierte, äquivalente Ligand als besser bindend betrachtet werden kann. Nichtsdestotrotz, liegen die ermittelten Werte im gleichen Größenbereich. Die strukturelle Ähnlichkeit von ProM-1 und ProM-4 drückt sich somit auch in den Affinitäten ihrer Liganden zur Domäne aus. Bemerkenswerte Resultate lieferten die Bindungsstudien zum ringmethylierten Liganden Ac-Phe(2-Cl)-[ProM-2][ProM-9]-OH. Die Einführung einer einzigen Methylgruppe (+14 Da) in den bisher bestbindenden Liganden Ac-Phe(2-Cl)-[ProM-2][ProM-1]-OH lieferte eine bis zu zehnfach verbesserte Bindungsaffinität.

Eine weitere Analyse wurde mittels FT und ITC durchgeführt, um die Dissoziationskonstante (Kd) verschiedener weitere Verbindungen zu testen. Insbesondere zeigte ProM-13, gekoppelt mit ProM-2, eine sehr hohe Affinität zu verschiedenen Targetmolekülen.

**Tabelle 2: Bindungsaffinitäten der verschiedenen Liganden bezüglich der VASP-EVH-1-, EnaH-EVH-1- und EVL-EVH-1-Domäne.**

| Scaffold: Ac[2-Cl-F][ProM-2][X]-OEt | | | |
|---|---|---|---|
| | X: ProM-1 | X: ProM-9 | X: ProM-13 |
| **VASP-EVH1** | | | |
| K_{D}, FT [µM] | 6.2 (0.6) | 0.78 (0.09) | 0.4 (0.1) |
| K_{D}, ITC [µM] | 9.4 (0.5) | 1.4 (0.2) | 0.49 (0.06) |

| **EnaH-EVH1** | | | |
|---|---|---|---|
| K_{D}, FT [µM] | 4.1 (0.3) | 0.38 (0.05) | 0.18 (0.03) |
| K_{D}, ITC [µM] | 7.8 (0.4) | 0.8 (0.1) | 0.31 (0.04) |

| **EVL-EVH1** | | | |
|---|---|---|---|
| K_{D}, FT [µM] | 4.1 (0.5) | 0.28 (0.05) | 0.13 (0.02) |
| K_{D}, ITC [µM] | 5.8 (0.7) | 0.67 (0.07) | 0.28 (0.03) |

Durch rationales Design, stereoselektive Synthese und biologische Tests konnte somit der erste, im (höheren) nanomolaren Bereich an EVH1-Domänen bindende Ligand entwickelt werden, welcher aufgrund seiner Masse (z. B. von 692 Da; mit Einschränkungen) in den niedermolekularen Substanzbereich eingeordnet werden kann.

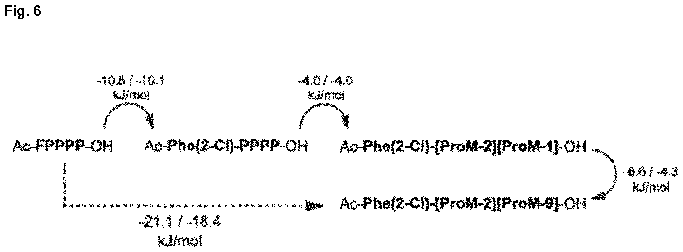

Mit dem nachfolgenden Fig. 6 soll ein Überblick über die Optimierung der Bindungsaffinitäten durch synthetische Veränderung des "wild-typ"-Liganden gegeben werden. Der Gesamtverlauf der Optimierung des Liganden über mehrere Generationen zu Ac-Phe(2-Cl)-[ProM-2][ProM-9]-OH war somit mit einem Enthalpiegewinn von ΔΔG = -15.9 kJ/mol (bestimmt mittels ITC) verbunden.

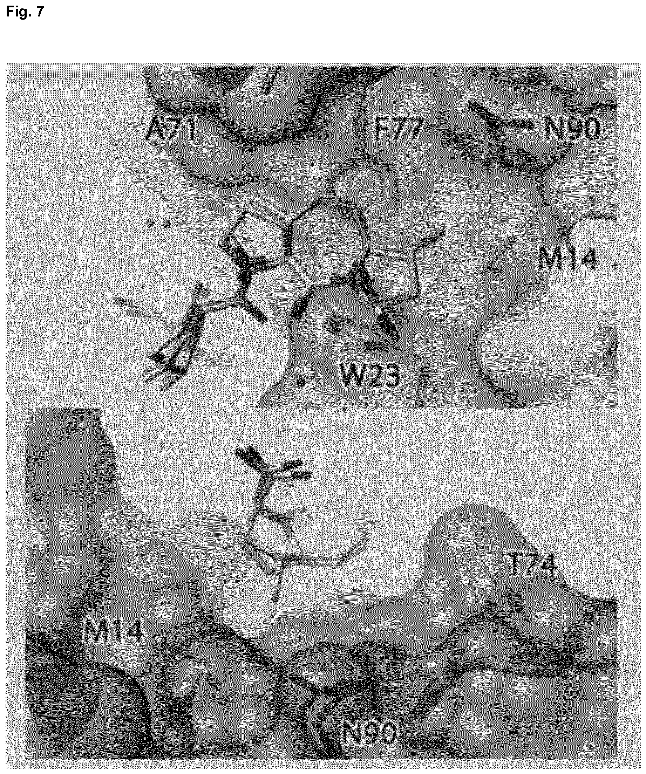

Die zusätzliche Methylgruppe in Ac-Phe(2-Cl)-[ProM-2][ProM-9]-OH hat mit Blick auf die erhaltenen Affinitätsergebnisse eine Bindungsverbesserung zur Folge. Der Effekt der Ligandensubstitution konnte zudem anhand von Kristallstrukturen des Liganden-Domänen Komplexes bewiesen werden. So gelang es Kristalle von Ac-Phe(2-Cl)-[ProM-2][ProM-1]-OH und Ac-Phe(2-Cl)-[ProM-2][ProM-9]-OH im Komplex mit der Proteinoberfläche einer EVH1-Domäne zu erhalten und so Rückschlüsse auf die strukturelle bzw. konformationelle Bindungssituation zu ziehen. In Fig. 7 ist eine Überlagerung der beiden Liganden an der Proteinoberfläche dargestellt.

Vergleicht man beide Liganden in ihrer Ausrichtung an der Proteinoberfläche, so ist festzustellen, dass die tricyclischen Strukturen (ProM-9 ↔ ProM-1), wie zu erwarten, nahezu identisch liegen. Im Sinne der konzeptionellen Idee deckt die R-konfigurierte Methylgruppe an Cy die hydrophobe Oberfläche des Proteins unterhalb des Prolinrrings zusätzlich ab, ohne dabei mit Methionin(M)-14 oder Asparagin(N)-90 zu kollidieren.

Des Weiteren konnte im Rahmen von Zellexperimenten gezeigt werden, dass durch den Ersatz des Carbonsäureliganden durch das Esterderivat (NBD-Phe(2-Cl)-[ProM-2][ProM-1]-OEt) die Fähigkeit der Verbindung, von den Targetzellen aufgenommen zu werden, verbessert werden konnte. Die Aufnahme der Substanz in die Zelle konnte somit gesteigert werden, ohne Verlust auf die vorteilhaften Bindungseigenschaften.

Beispielhaft zeigt folgende Verbindung III eine unerwartete Kombination von vorteilhaften Eigenschaften:

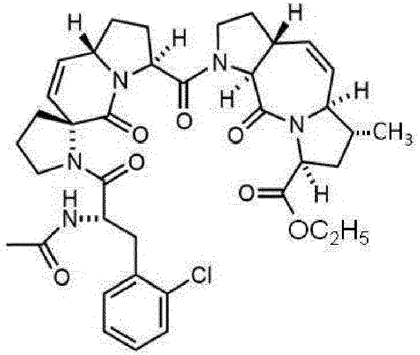

Diese Verbindung (gemäß Formel III), und nach weiteren Untersuchungen auch die Verbindungen gemäß den Formeln IV, V, VI, VII und VIII, hemmen selektiv ena/VASP- EVH1-vermittelte Protein-Protein-Wechselwirkungen. Die Bindungsdaten für Verbindung III an Ena/VASP-EVH1 sind: VASP-EVH1 Kd 280 nM (Faktor 100 besser als das Referenzpeptid (SFEFPPPPTEDEL Kd 20 µM). Dies bedeutet, dass das Esterderivat an der Position R3 bzw. R6 gemäß der allgemeinen Formel I nicht nur eine verbesserte Zellpermeabilität sondern auch verbesserte Bindungseigenschaften aufweist. Es konnte weiterhin beobachtet werden, dass die Kombination Ac-Phe(2-Cl)-[ProM-2][ProM-13]-OEt eine besonders starke Affinität zum Target aufweist.

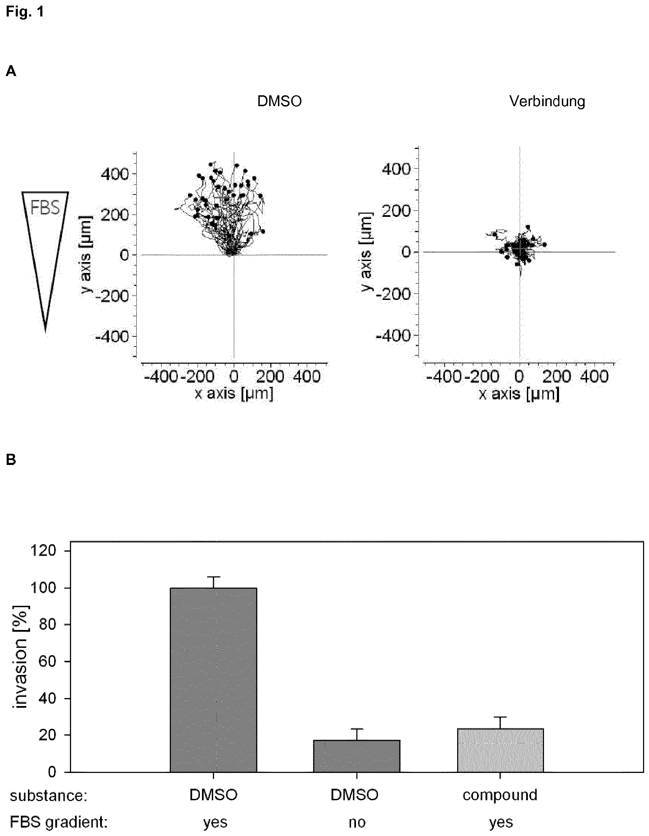

Die Verbindungen gemäß den Formeln III-VIII hemmen überraschenderweise die Chemotaxis und Motilität hoch invasiver Tumorzellen. Zellkultur-Experimente wurden mit MDA MB 231 Brustkrebszellen durchgeführt. In einem Matrigel-Migrationsassay konnte gezeigt werden, dass die Beispielverbindung III die Migration von MDA MB 231 Brustkrebszellen um mehr als 90%, bezogen auf den Basalwert (DMSO/kein FBS-Gradient) hemmt (Fig. 1A und B).

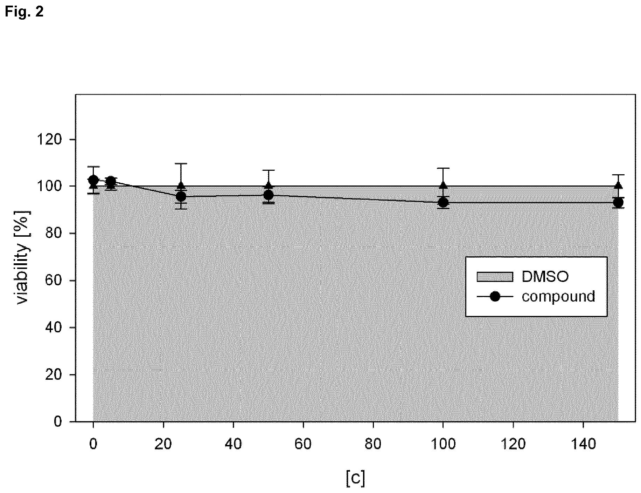

Die Verbindung erwies sich dabei als nicht zelltoxisch (Fig. 2). Die geringe bzw. abwesende Zelltoxizität stellt ebenfalls eine überraschende und vorteilhafte Eigenschaft der Verbindungen dar. Die erfindungsgemäßen Verbindungen binden nicht nur mit sehr niedrigen Dissoziationskonstanten an den Targetrezeptor, sondern auch mit einer hohen Spezifität. Dies ist für eine medizinische Anwendung der Verbindungen als Vorteil anzusehen.

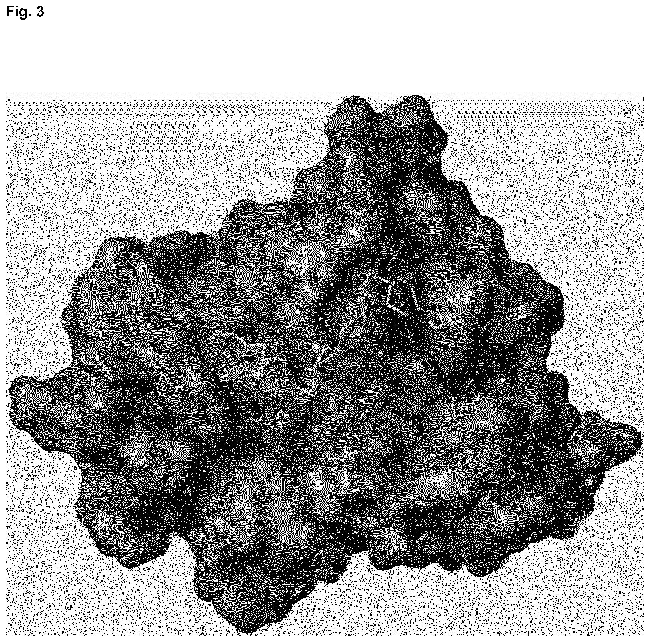

Die Bindung des Liganden an ena-EVH1 konnte über Kristallisation des Komplexes mit Ena-EVH1 (Auflösung 1.02Å) nachgewiesen werden (Fig. 3). Hierdurch wird bewiesen, dass die beanspruchten Verbindungen außergewöhnlich gute Bindungseigenschaften aufweisen.

Die erfindungsgemäßen Verbindungen wurden weiterhin bezüglich ihrer Stabilität und Protein-Bindungseigenschaften in Plasma, sowie ihrer metabolischen Stabilität, getestet. Überraschenderweise zeigen die erfindungsgemäßen Verbindungen eine außergewöhnlich gute Stabilität in Plasma, eine niedrige Proteinbindung in Plasma (nur wenig unspezifische ProteinBindung) und eine hohe metabolische Stabilität, die für eine klinische Anwendung als vorteilhaft anzusehen ist.

Die Verbindungen Ac-Phe(2-Cl)-[ProM-2][ProM-1]-OEt (nachfolgend als Verbindung 4D benannt) und Ac-Phe(2-Cl)-[ProM-2][ProM-1]-NH₂ (nachfolgend als Verbindung 4C benannt) wurden untersucht.

Ein in vitro-Assay wurde im Plasma von Mäusen und Menschen durchgeführt. Insgesamt war 4C in Maus- und Humanplasma sehr stabil (94,7% und 80,3% verbliebene Verbindung nach 240 Minuten Inkubation). Für 4D wurden 87,4% und 95,5% verbliebene Verbindung gemessen, was seine hohe Stabilität im Plasma von Mäusen und Menschen zeigt (Halbwertszeit> 240 min).

Um die Plasmaproteinbindung von Verbindungen 4C und 4D zu beurteilen, wurde eine modifizierte Ultrafiltrationstechnik angewandt. 4C zeigte eine sehr niedrige Affinität für Plasmaproteine, was zu PPB-Werten von 5,5% und 4,5% (Mensch und Maus) führte. Eine geringe Plasmaproteinbindung wurde auch für 4D beobachtet (1,9% und 7,6% mit Maus und menschlichem Plasma).

Die metabolische Stabilität der 4C und 4D wurde mit gepoolten Maus (MLM) und humanen Lebermikrosomen (HLM) getestet. Mit MLM und HLM wurden 4C und 4D langsam metabolisiert, was zu Clᵢₙₜ-Werten im Bereich von 34,0 und 35,6 µl/min/mg Protein mit MLM und 11,6 und 26,4 µl/min/mg Protein mit HLM führte.

**Tabelle 3: Plasmastabilität, unspezifische Proteinbindung in Plasma und metabolische Stabilität der Verbindungen 4C und 4D.**

| Assay | Spezies | Verbindung | Parameter | Ergebnis | Bemerkung |
|---|---|---|---|---|---|
| Plasmastabilität | Maus | 4C | % verbleibend (Tlast 240 min) | 94.7 | Hohe Stabilität in Plasma |
| | Human | | | 80.3 | |
| | Maus | 4D | | 87.4 | |
| | Human | | | 95.5 | |
| PlasmaProteinbindung | Maus | 4C | fb (gebundene Fraktion) | 5.5 | Low PPB |
| | Human | | | 4.5 | |
| | Maus | 4D | | 1.9 | |
| | Human | | | 7.6 | |
| Leber mikrosomale Stabilität | Maus, Human | 4C | t1/2 [min] | > 60 | High stability regarding Phase I metabolism |
| | | 4D | | > 60 | |
| | Maus | 4C | Clint [µl/min/ mg Protein] | 34.0 | |
| | Human | | | 11.6 | |
| | Maus | 4D | | 35.6 | |
| | Human | | | 26.4 | |

## Patentansprüche

1. Verbindung gemäß Formel I: 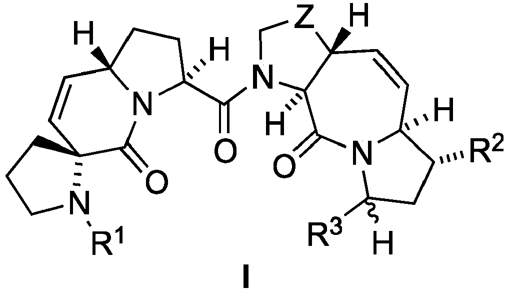 wobei
R¹: H, Acyl, Peptidyl, Sulfonyl, Alkyl, Aryl oder Heteroaryl ist, wobei die Acyl, Peptidyl, Sulfonyl, Alkyl, Aryl oder Heteroaryl Substituenten gemäß R¹ optional substituiert sind;
R²_{:} CH₃ oder C₂H₅;
R³: Alkyl, Acyl, Carbonsäure gemäß C(=O)X, wobei X = OH, O-Alkyl, O-Aryl, NRR', mit R, R' = H oder Alkyl, NROR', mit R, R' = H oder Alkyl, oder Heteroaryl;
Z: CH₂ oder CH₂CH₂ ist.

2. Verbindung nach Anspruch 1 gemäß Formel I-a: 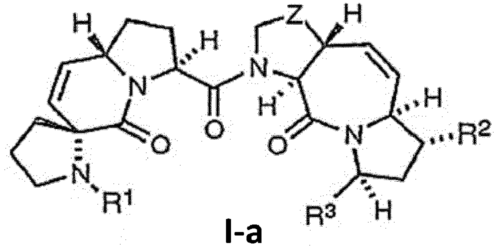 wobei R¹, R², R³ und Z sind gemäß Anspruch 1.

3. Verbindung nach Anspruch 1 oder 2 gemäß Formel II: 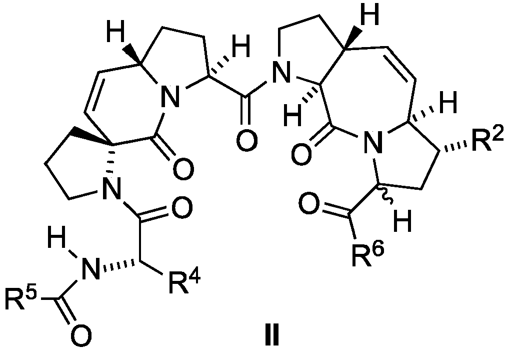 wobei
R²: CH₃ oder C₂H₅;
R⁴: H, Alkyl, Cycloalkyl, Aryl, CH₂-Aryl, CH₂-Heteroaryl, oder ein heterozyklischer Substituent;
R⁵: H, Alkyl oder ein substituierter Alkylrest;
R⁶: OH, O-Alkyl, O-Aryl, NRR' mit R, R' = H oder Alkyl, NROR' mit R, R' = H oder Alkyl,
oder Heteroaryl.

4. Verbindung nach einem der vorhergehenden Ansprüche gemäß Formel II-a: 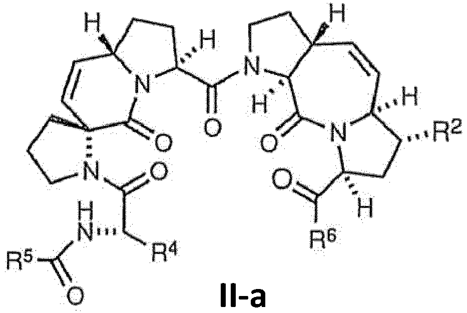 wobei R², R⁴, R⁵ und R⁶ sind gemäß Anspruch 3.

5. Verbindung nach Anspruch 3 oder 4 gemäß Formel II oder II-a, wobei R⁴: substituiertes - CH₂-Phenyl ist, wobei der Substituent an der Ortho-Position der Phenylgruppe positioniert ist, wobei der Substituent Halogen ist.

6. Verbindung nach Anspruch 3 oder 4 gemäß Formel II oder II-a, wobei R4: -CH₂-3-Indolyl ist.

7. Verbindung nach Anspruch 3 oder 4 gemäß Formel II oder II-a, wobei R⁶: O-Alkyl, O-Aryl, NRR' mit R, R' = H oder Alkyl, NROR' mit R, R' = H oder Alkyl, oder Heteroaryl ist.

8. Verbindung nach einem der vorhergehenden Ansprüche gemäß Formel III-VIII:

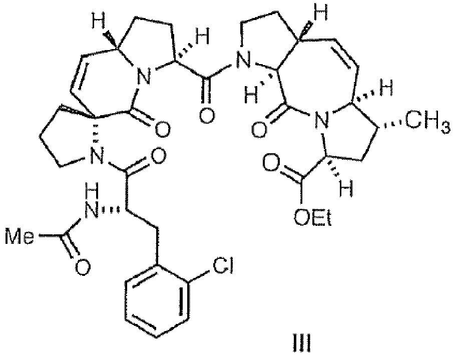

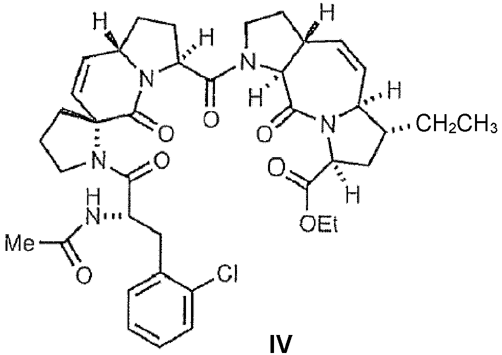

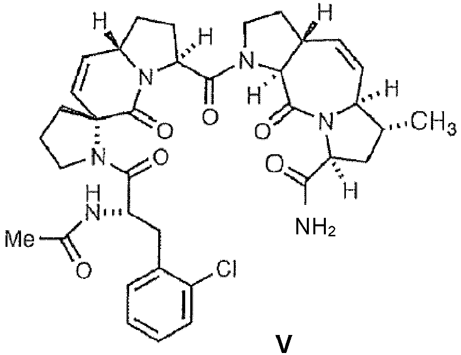

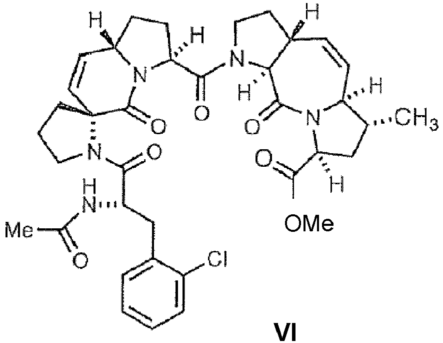

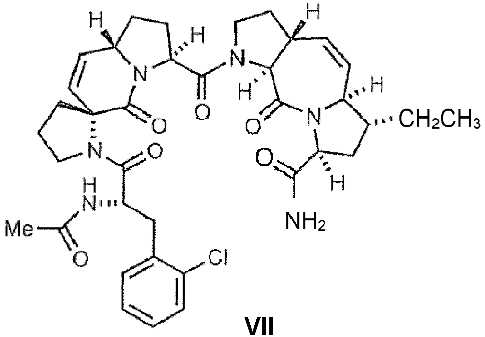

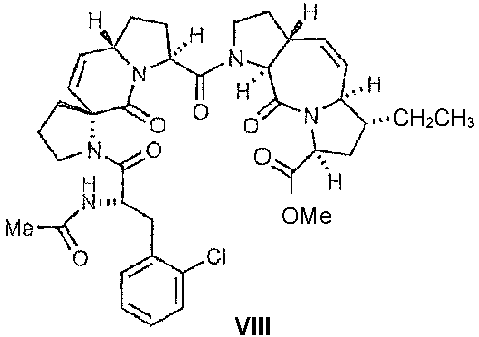

9. Pharmazeutische Zusammensetzung umfassend eine Verbindung nach einem der vorhergehenden Ansprüche, mit einem pharmazeutisch annehmbaren Träger.

10. Verbindung oder Zusammensetzung nach einem der vorhergehenden Ansprüche zur Verwendung als Medikament zur Behandlung von Tumorerkrankungen.

11. Verbindung oder Zusammensetzung zur Verwendung als Medikament nach dem vorhergehenden Anspruch, wobei die Tumorerkrankung Brustkrebs ist.

12. Verbindung oder Zusammensetzung zur Verwendung als Medikament nach Anspruch 10 zur Behandlung von Tumorerkrankungen, **dadurch gekennzeichnet, dass** die Verbindung die Metastasierung eines Tumors inhibiert.

13. Verbindung oder Zusammensetzung nach dem vorhergehenden Anspruch zur Verwendung als Medikament zur Behandlung von Tumorerkrankungen, **dadurch gekennzeichnet, dass** die Metastasierung eines Tumors durch Inhibition der Chemotaxis und/oder Motilität der Tumorzellen auf Basis einer Störung der Aktinfilamentsynthese inhibiert wird.

## Claims

1. A compound according to formula I: 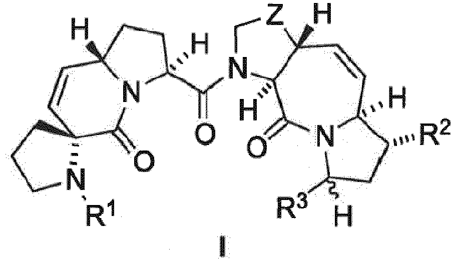 wherein
R¹: is H, acyl, peptidyl, sulfonyl, alkyl, aryl or heteroaryl, wherein the acyl, peptidyl, sulfonyl, alkyl, aryl or heteroaryl substituents according to R¹ are optionally substituted;
R²: CH₃ or C₂H₅;
R³: is alkyl, acyl, carboxylic acid according to C(=O)X, wherein X = OH, O-alkyl, O-aryl, NRR', with R, R' = H or alkyl, NROR', with R, R' = H or alkyl, or heteroaryl;
Z: is CH₂ or CH₂CH₂.

2. The compound according to claim 1 according to formula I-a: 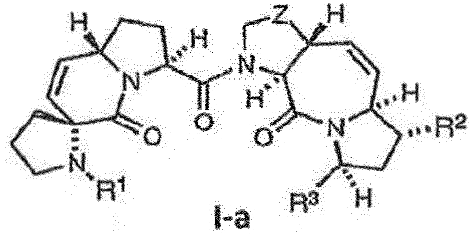 wherein R¹, R², R³ and Z are according to claim 1.

3. The compound according to claim 1 or 2 according to formula II: 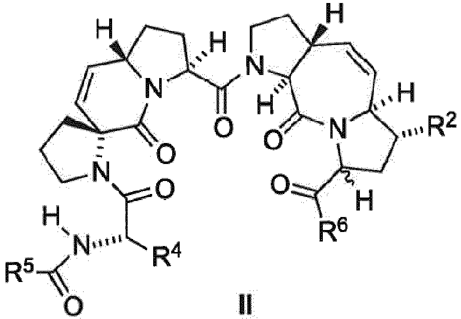 wherein
R²: CH₃ or C₂H₅;
R⁴: H, alkyl, cycloalkyl, aryl, CH₂-aryl, CH₂-heteroaryl, or a heterocyclic substituent;
R⁵: H, alkyl or a substituted alkyl radical;
R⁶: OH, O-alkyl, O-aryl, NRR' with R, R' = H or alkyl, NROR' with R, R' = H or alkyl, or heteroaryl.

4. The compound according to any one of the preceding claims according to formula II-a: 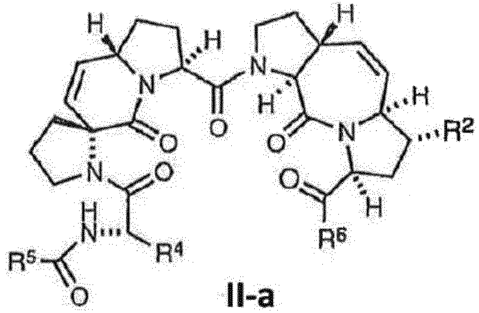 wherein R², R⁴, R⁵ and R⁶ are according to claim 3.

5. The compound according to claim 3 or 4 according to formula II or II-a, wherein R⁴: is substituted -CH₂-phenyl, wherein the substituent is positioned at the ortho position of the phenyl group, wherein the substituent is halogen.

6. The compound according to claim 3 or 4 according to formula II or II-a, wherein R4: is -CH₂-3-indolyl.

7. The compound according to claim 3 or 4 according to formula II or II-a, wherein R⁶: is O-alkyl, O-aryl, NRR' with R, R' = H or alkyl, NROR' with R, R' = H or alkyl, or heteroaryl.

8. The compound according to any one of the preceding claims according to formula III-VIII:

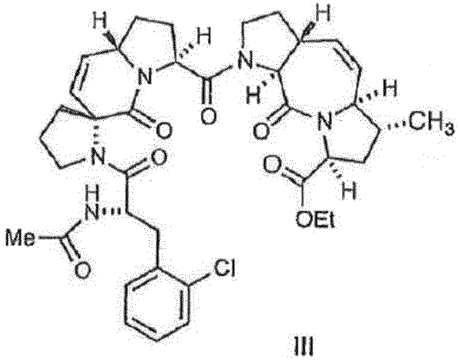

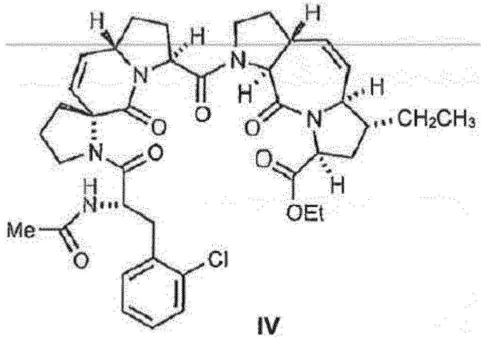

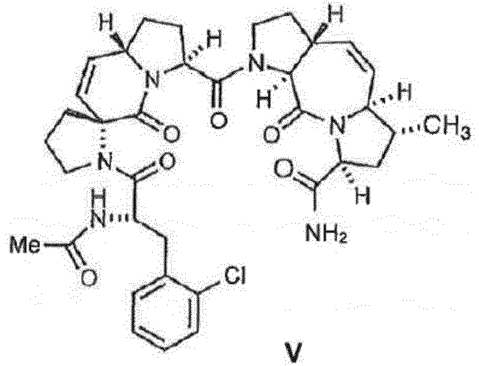

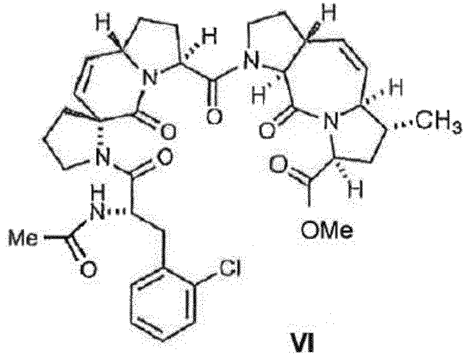

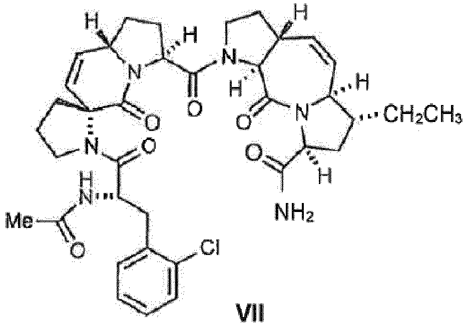

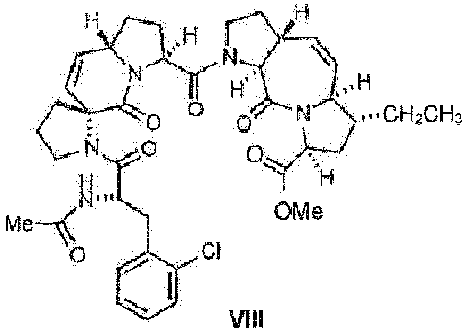

9. A pharmaceutical composition comprising a compound according to any one of the preceding claims, with a pharmaceutically acceptable carrier.

10. The compound or composition according to any one of the preceding claims for use as a medicament for the treatment of tumor diseases.

11. The compound or composition for use as a medicament according to the preceding claim, wherein the tumor disease is breast cancer.

12. The compound or composition for use as a medicament according to claim 10 for the treatment of tumor diseases, **characterized in that** the compound inhibits the metastasis of a tumor.

13. The compound or composition according to the preceding claim for use as a medicament for the treatment of tumor diseases, **characterized in that** the metastasis of a tumor is inhibited by inhibition of chemotaxis and/or motility of the tumor cells based on interference with the actin filament synthesis.

## Revendications

1. Composé de formule I : 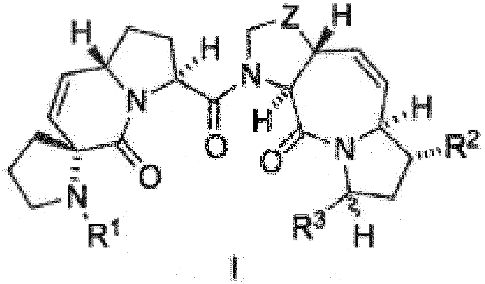 dans lequel :
R¹ désigne H, les groupes acyle, peptidyle, sulfonyle, alkyle, aryle ou hétéroaryle, dans lequel les substituants acyle, peptidyle, sulfonyle, alkyle, aryle ou hétéroaryle sont substitués facultativement selon R¹;
R² désigne le groupe CH₃ ou C₂H₅;
R³ désigne les groupes alkyle, acyle, acide carboxylique selon C(=O)X, où X = OH, O-alkyle, O-aryle, NRR', avec R, R'= H ou alkyle, NROR', avec R, R'=H ou alkyle ou hétéroaryle;
Z désigne le groupe CH₂ ou CH₂CH₂.

2. Composé selon la revendication 1 de formule I-a : 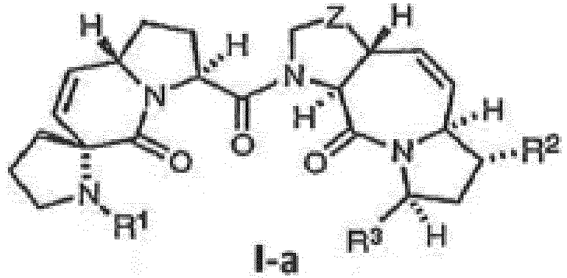 dans lequel R¹, R², R³ et Z sont désignés selon la revendication 1.

3. Composé selon la revendication 1 ou 2 de formule II : 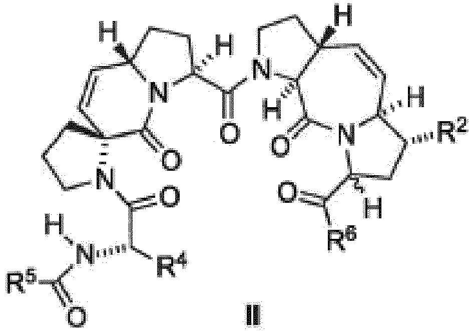 dans lequel :
R² désigne le groupe CH₃ ou C₂H₅;
R⁴ désigne H, les groupes alkyle, cycloalkyle, aryle, CH₂-aryle, CH₂-hetéroaryle ou un substituant hétérocyclique ;
R⁵ désigne H, un groupe alkyle ou un radical alkyle substitué ;
R⁶ désigne les groupes OH, O-alkyle, O-aryle, NRR' avec R, R' = H ou un groupe alkyle, NROR' avec R, R' = H ou un groupe alkyle ou hétéroaryle.

4. Composé selon l'une quelconque des revendications précédentes de formule II-a : 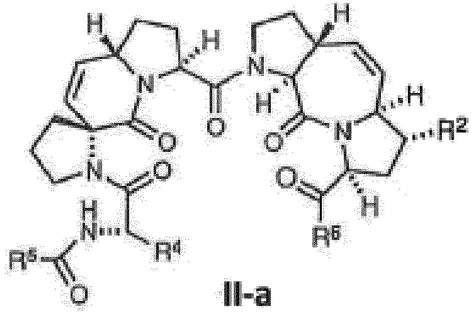 dans lequel R², R⁴, R⁵, et R⁶ sont conformes à la revendication 3.

5. Composé selon la revendication 3 ou 4 de formule II ou II-a, dans lequel R⁴ désigne un groupe CH₂-phényle substitué, dans lequel le substituant est positionné dans la position ortho du groupe phényle, dans lequel le substituant est un halogène.

6. Composé selon la revendication 3 ou 4 de formule II ou II-a, dans lequel R4 désigne un groupe -CH₂-3-indolyle.

7. Composé selon la revendication 3 ou 4 de formule II ou II-a, dans lequel R⁶ désigne un groupe O-alkyle, O-aryle, NRR' avec R, R' = H ou alkyle, NROR' avec R, R' = H ou alkyle, ou hétéroaryle.

8. Composé selon l'une quelconque des revendications précédentes de formules III-VIII :

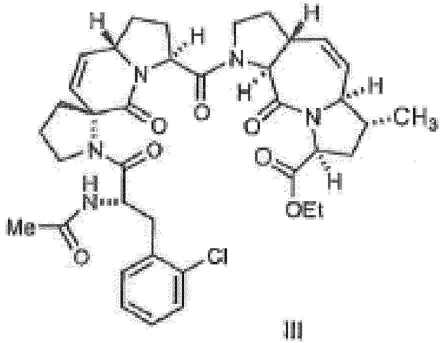

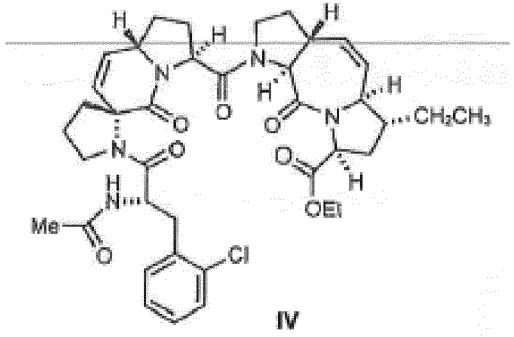

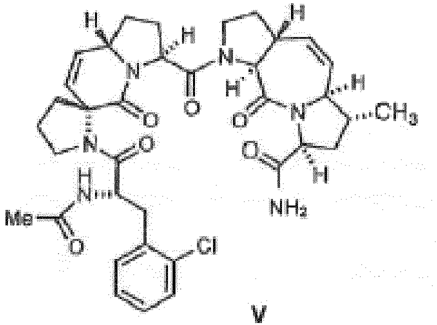

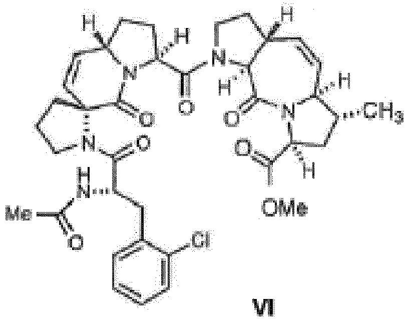

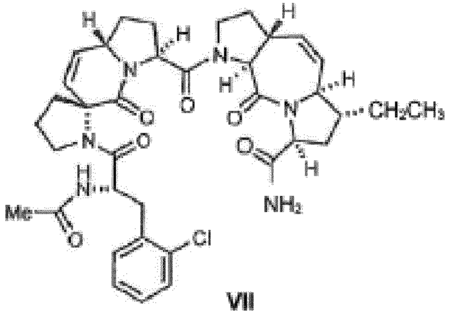

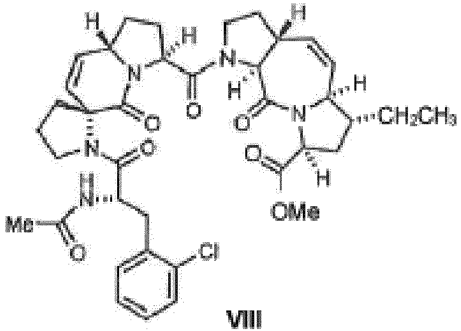

9. Composition pharmaceutique comprenant un composé selon l'une quelconque des revendications précédentes avec un support pharmaceutiquement acceptable.

10. Composé ou composition selon l'une quelconque des revendications précédentes pour utilisation comme médicament pour le traitement de maladies tumorales.

11. Composé ou composition pour utilisation comme médicament selon la revendication précédente, dans lequel ou laquelle la maladie tumorale est un cancer du sein.

12. Composé ou composition pour utilisation comme médicament selon la revendication 10 pour le traitement de maladies tumorales, caractérisé(e) en ce que le composé inhibe la métastase d'une tumeur.

13. Composé ou composition selon la revendication précédente pour utilisation comme médicament pour le traitement de maladies tumorales, caractérisé(e) en ce que la métastase d'une tumeur est inhibée par inhibition de la chimiotaxie et/ou de la motilité des cellules tumorales sur la base d'une perturbation de la synthèse de filaments d'actine.
